(19) Europäisches Patentamt
European Patent Office
Office européen des brevets

(11) Veröffentlichungsnummer: **0 472 077 A2**

(12) # EUROPÄISCHE PATENTANMELDUNG

(21) Anmeldenummer: **91113482.3**

(22) Anmeldetag: **12.08.91**

(51) Int. Cl.[5]: **C07K 5/02**, C07K 5/06, C07F 9/572, C07F 9/59, C07D 303/36, C07D 331/02, A61K 37/64, A61K 31/675, A61K 31/335, A61K 31/38

(30) Priorität: **24.08.90 DE 4026703**
**01.11.90 DE 4034707**

(43) Veröffentlichungstag der Anmeldung:
**26.02.92 Patentblatt 92/09**

(84) Benannte Vertragsstaaten:
**AT BE CH DE DK ES FR GB GR IT LI LU NL SE**

(71) Anmelder: **BAYER AG**

**W-5090 Leverkusen 1 Bayerwerk(DE)**

(72) Erfinder: **Häbich, Dieter, Dr.**
**Krummacherstrasse 82**
**W-5600 Wuppertal(DE)**
Erfinder: **Hansen, Jutta, Dr.**
**Claudiusweg 9**
**W-5600 Wuppertal(DE)**
Erfinder: **Paessens, Arnold, Dr.**
**Stresemannstrasse 51**
**W-5657 Haan(DE)**

(54) **Phosphonat-haltige Pseudopeptide vom Hydroxyethylamin- und Norstatin-Typ.**

(57) Die Erfindung betrifft phosphonat-haltige Pseudopeptide vom Hydroxyethylamin- und Norstatin-Typ, Verfahren zu ihrer Herstellung und ihre Verwendung als retrovirale Mittel.

EP 0 472 077 A2

Die Erfindung betrifft phosphonat-haltige Pseudopeptide vom Hydroxyethylamin- und Norstatin-Typ, neue Oxiran- und Thiiran-haltige Pseudopeptide als Zwischenprodukte, Verfahren zu ihrer Herstellung und ihre Verwendung als retrovirale Mittel.

Es wurde schon versucht, Pseudopeptide, die teilweise auch renininhibitorisch wirksam sind, bei der Bekämpfung von AIDS einzusetzen [vgl. GB A2 203 740; EP 337 714; EP 342 541; EP 346 847 und EP 352 000; EP 354 522; EP 356 223; EP 357 332; EP 337 714 und EP 357 332].

Außerdem ist bekannt, daß einfache Epoxide wie EPNP [1,2-Epoxy-3-(4-nitrophenoxy)propan] eine schwache Inaktivierung von HIV-I Protease ($IC_{50}$ = 450 $\mu$M) bewirken [vgl. T.D. Meek et al., in V. Kostha (Editors) "Protease of Retroviruses" Proceedings of the Colloquium C52 14th International Congress of Biochemistry, Prague, Czechoslovakia July 10 - 15 (1988), Walter de Gruyter 1989]. Ein Hinweis auf Peptide ist darin nicht gegeben.

Die vorliegende Erfindung betrifft neue phosphonat-haltige Pseudopeptide vom Hydroxyethylamin- und Norstatin-Typ der allgemeinen Formel (I),

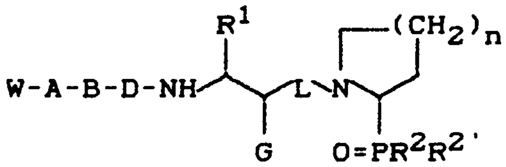

(I)

in welcher

W

- für Wasserstoff oder für eine typische Aminoschutzgruppe steht, oder
- für geradkettiges oder verzweigtes Alkyl oder Alkenyl mit jeweils bis zu 6 Kohlenstoffatomen steht, die gegebenenfalls durch Aryl mit 6 bis 10 Kohlenstoffatomen substituiert sind, oder
- für eine Gruppe der Formel $R^3$-CO-, $R^5R^4$N-CO- oder $R^6$-$SO_2$- steht,

  worin

  $R^3$

  - Wasserstoff, Trifluormethyl oder geradkettiges oder verzweigtes Alkoxy mit bis zu 8 Kohlenstoffatomen oder Alkyl mit bis zu 18 Kohlenstoffatomen bedeutet, das gegebenenfalls bis zu 2-fach durch Aryl mit 6 bis 10 Kohlenstoffatomen oder Pyridyl substituiert ist, oder
  - Aryl mit 6 bis 10 Kohlenstoffatomen bedeutet, das gegebenenfalls durch Halogen, Trifluormethyl, Trifluormethoxy oder durch geradkettiges oder verzweigtes Alkyl mit bis zu 8 Kohlenstoffatomen substituiert ist,
  - Cycloalkyl mit 3 bis 7 Kohlenstoffatomen bedeutet, oder
  - Chinolyl, Chinolyl-N-oxid, Indolyl, Pyridyl, Pyridyl-N-oxid, Morpholino oder Piperazinyl bedeutet, oder
  - einen Rest der Formel

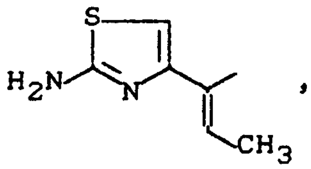 ,

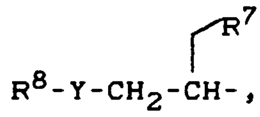 ,

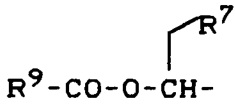 ,

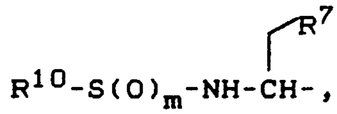 ,

T-NH-$(CH_2)_p$- ,

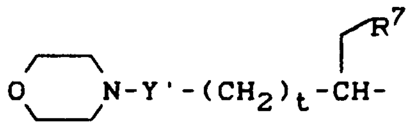

oder

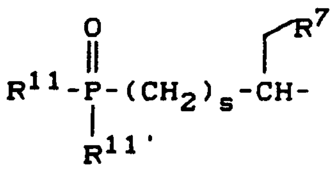

bedeutet,
worin
$R^7$ - Phenyl oder Naphthyl bedeutet,
$R^8$, $R^9$ und $R^{10}$ gleich oder verschieden sind und geradkettiges oder verzweigtes Alkyl mit bis zu 14 Kohlenstoffatomen bedeuten, das gegebenenfalls durch Phenyl oder Naphthyl substituiert ist, oder Aryl mit 6 bis 10 Kohlenstoffatomen bedeuten, welches seinerseits durch Alkyl mit bis zu 4 Kohlenstoffatomen substituiert ist,
m - eine Zahl 0, 1 oder 2 bedeutet,
T - Cyclohexyl bedeutet,
p - eine Zahl 1, 2 oder 3 bedeutet,
Y und Y' gleich oder verschieden sind und die CO- oder $SO_2$-Gruppe bedeuten,
t - eine Zahl 0 oder 1 bedeutet,
$R^{11}$ und $R^{11'}$ gleich oder verschieden sind und Hydroxy oder Alkoxy mit bis zu 8 Kohlenstoffatomen bedeuten,
s - eine Zahl 1 oder 2 bedeutet,

$R^4$ und $R^5$ gleich oder verschieden sind und
- Wasserstoff oder
- Aryl mit 6 bis 10 Kohlenstoffatomen bedeuten, das gegebenenfalls durch geradkettiges oder verzweigtes Alkyl mit bis zu 6 Kohlenstoffatomen oder Halogen substituiert ist, oder
- Cycloalkyl mit 3 bis 7 Kohlenstoffatomen bedeuten, oder
- geradkettiges oder verzweigtes Alkyl mit bis zu 18 Kohlenstoffatomen bedeuten,

$R^6$ - geradkettiges oder verzweigtes Alkyl mit bis zu 8 Kohlenstoffatomen bedeutet,

A, B und D gleich oder verschieden sind und
- für eine direkte Bindung oder
- für einen Rest der Formel

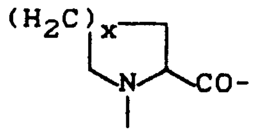 oder 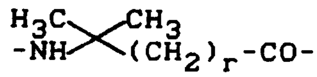

stehen,
worin
x - die Zahl 1 oder 2 bedeutet
und
r - die Zahl 0 oder 1 bedeutet,
oder

- für eine Gruppe der Formel

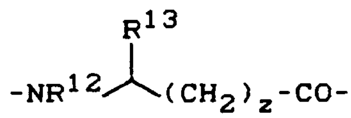

stehen
worin
z - die Zahl 0 oder 1 bedeutet,
$R^{12}$ Wasserstoff oder geradkettiges oder verzweigtes Alkyl mit bis zu 4 Kohlenstoffatomen bedeutet,
$R^{13}$ Cycloalkyl mit 3 bis 8 Kohlenstoffatomen oder Aryl mit 6 bis 10 Kohlenstoffatomen oder Wasserstoff bedeutet, oder

- geradkettiges oder verzweigtes Alkyl mit bis zu 8 Kohlenstoffatomen bedeutet, das gegebenenfalls durch Methylthio, Hydroxy, Mercapto, Guanidyl oder durch eine Gruppe der Formel $-NR^{14}R^{15}$ oder $R^{16}$-OC-substituiert ist,
  worin
  $R^{14}$ und $R^{15}$ gleich oder verschieden sind und Wasserstoff, geradkettiges oder verzweigtes Alkyl mit bis zu 8 Kohlenstoffatomen oder Phenyl bedeuten
  und
  $R^{16}$ Hydroxy, Benzyloxy, Alkoxy mit bis zu 6 Kohlenstoffatomen oder die oben aufgeführte Gruppe $-NR^{14}R^{15}$ bedeutet,

oder das gegebenenfalls durch Cycloalkyl mit 3 bis 8 Kohlenstoffatomen oder durch Aryl mit 6 bis 10 Kohlenstoffatomen substituiert ist, das seinerseits durch Hydroxy, Halogen, Nitro, Alkoxy mit bis zu 8 Kohlenstoffatomen oder durch die Gruppe $-NR^{14}R^{15}$ substituiert ist,
worin
$R^{14}$ und $R^{15}$ die oben angegebene Bedeutung haben,
oder das gegebenenfalls durch einen 5- oder 6-gliedrigen stickstoffhaltigen Heterocyclus oder Indolyl substituiert ist, worin die entsprechenden -NH-Funktionen gegebenenfalls durch Alkyl mit bis zu 6 Kohlenstoffatomen oder durch eine Aminoschutzgruppe geschützt sind,

$R^1$ - für geradkettiges oder verzweigtes Alkyl oder Alkenyl mit bis zu 10 Kohlenstoffatomen steht, die gegebenenfalls durch Cycloalkyl mit 3 bis 8 Kohlenstoffatomen oder Aryl mit 6 bis 10 Kohlenstoffatomen substituiert sind, das seinerseits durch Halogen, Nitro, Hydroxy, Amino oder geradkettiges oder verzweigtes Alkoxy mit bis zu 4 Kohlenstoffatomen substituiert sein kann,

n - für die Zahl 1 oder 2 steht,

$R^2$ und $R^{2'}$ gleich oder verschieden sind

- für Hydroxy, oder
- für geradkettiges oder verzweigtes Alkoxy mit bis zu 4 Kohlenstoffatomen stehen,

G für eine Gruppe der Formel -SH oder -OH steht,
und

L - für die $-CH_2$ oder -CO-Gruppe steht
und deren physiologisch unbedenklichen Salze.

Die erfindungsgemäßen Verbindungen der allgemeinen Formel (I), haben mehrere asymmetrische Kohlenstoffatome. Sie können unabhängig voneinander in der D- oder der L-Form vorliegen. Die Erfindung umfaßt die optischen Antipoden ebenso wie die Isomerengemische oder Racemate. Bevorzugt liegen die Gruppen A, B und D unabhängig voneinander in der optisch reinen, bevorzugt in der L-Form vor.

Der Rest der allgemeinen Formel (XII)

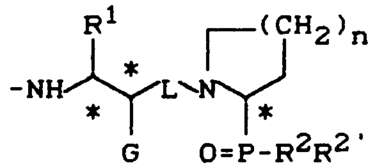

(XII)

besitzt 3 asymmetrische Kohlenstoffatome (*), die unabhängig voneinander in der R- oder S-Konfiguration vorliegen können.

Aminoschutzgruppen im Rahmen der Erfindung sind die üblichen in der Peptid-Chemie verwendeten Aminoschutzgruppen.

Hierzu gehören bevorzugt: Benzyloxycarbonyl, 3,4-Dimethoxybenzyloxycarbonyl, 3,5-Dimethoxybenzyloxycarbonyl, 2,4-Dimethoxybenzyloxycarbonyl, 4-Methoxybenzyloxycarbonyl, 4-Nitrobenzyloxycarbonyl, 2-Nitrobenzyloxycarbonyl, 2-Nitro-4,5-dimethoxybenzyloxycarbonyl, Methoxycarbonyl, Ethoxycarbonyl, Propoxycarbonyl, Isopropoxycarbonyl, Butoxycarbonyl, Isobutoxycarbonyl, tert-Butoxycarbonyl, Allyloxycarbonyl, Vinyloxycarbonyl, 2-Nitrobenzyloxycarbonyl, 3,4,5-Trimethoxybenzyloxycarbonyl, Methoxycarbonyl, Ethoxycarbonyl, Propoxycarbonyl, Isopropoxycarbonyl, Butoxycarbonyl, Isobutoxycarbonyl, tert-Butoxycarbonyl, Cyclohexoxycarbonyl, 1,1-Dimethylethoxycarbonyl, Adamantylcarbonyl, Phthaloyl, 2,2,2-Trichlorethoxycarbonyl, 2,2,2-Trichlor-tert-butoxycarbonyl, Menthyloxycarbonyl, Phenoxycarbonyl, 4-Nitrophenoxycarbonyl, Fluorenyl-9-methoxycarbonyl, Formyl, Acetyl, Propionyl, Pivaloyl, 2-Chloracetyl, 2-Bromacetyl, 2,2,2-Trifluoracetyl, 2,2,2-Trichloracetyl, Benzoyl, 4-Chlorbenzoyl, 4-Brombenzoyl, 4-Nitrobenzoyl, Phthalimido, Isovaleroyl oder Benzyloxymethylen.

Die erfindungsgemäßen Verbindungen der allgemeinen Formel (I) können in Form ihrer Salze vorliegen. Dies können Salze mit anorganischen oder organischen Säuren oder Basen sein.

Bevorzugt sind Verbindungen der allgemeinen Formel (I),
in welcher

W
- für Wasserstoff, tert.Butyloxycarbonyl (BOC), 9-Fluorenylmethyloxycarbonyl (Fmoc) oder Benzyloxycarbonyl (Z) steht, oder
- für geradkettiges oder verzweigtes Alkyl oder Alkenyl mit jeweils bis zu 4 Kohlenstoffatomen steht, die gegebenenfalls durch Phenyl substituiert sind, oder
- für eine Gruppe der Formel $R^3$-CO-, oder $R^5R^4$N-CO- oder $R^6$-$SO_2$ steht,
  worin
  $R^3$
  - Wasserstoff, Trifluormethyl oder geradkettiges oder verzweigtes Alkoxy mit bis zu 4 Kohlenstoffatomen oder Alkyl mit bis zu 16 Kohlenstoffatomen bedeutet, das gegebenenfalls bis zu 2-fach durch Phenyl, Naphthyl oder Pyridyl substituiert ist, oder
  - Phenyl oder Naphthyl bedeutet, die gegebenenfalls durch Fluor, Chlor, Trifluormethyl, Trifluormethoxy oder durch geradkettiges oder verzweigtes Alkyl mit bis zu 6 Kohlenstoffatomen substituiert sind,
  - Cyclopropyl, Cyclopentyl, Cyclohexyl, Chinolyl-N-oxid, Chinolyl, Indolyl, Pyridyl, Pyridyl-N-oxid, Morpholino oder Piperazinyl bedeutet, oder
  - einen Rest der Formel

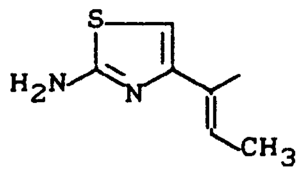 ,

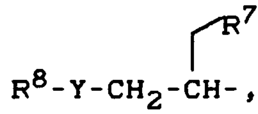 ,

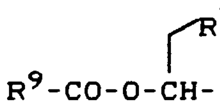 oder 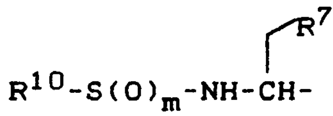

bedeutet,

worin

Y - die CO- oder $SO_2$-Gruppe bedeutet,

$R^7$ - Phenyl oder Naphthyl bedeutet,

$R^8$, $R^9$ und $R^{10}$ gleich oder verschieden sind und geradkettiges oder verzweigtes Alkyl mit bis zu 8 Kohlenstoffatomen, Tolyl, Phenyl oder Naphthyl bedeuten,

m - eine Zahl 1 oder 2 bedeutet,

$R^4$ und $R^5$ gleich oder verschieden sind und

- Wasserstoff oder
- Phenyl oder Naphthyl bedeuten, die gegebenenfalls durch geradkettiges oder verzweigtes Alkyl mit bis zu 4 Kohlenstoffatomen, Fluor oder Chlor substituiert sind,
- Cyclopropyl, Cyclopentyl oder Cyclohexyl bedeuten, oder
- geradkettiges oder verzweigtes Alkyl mit bis zu 16 Kohlenstoffatomen bedeuten,

$R^6$ - geradkettiges oder verzweigtes Alkyl mit bis zu 6 Kohlenstoffatomen bedeutet,

A, B und D gleich oder verschieden sind und

- für eine direkte Bindung oder
- für Prolin stehen, oder
- für einen Rest der Formel

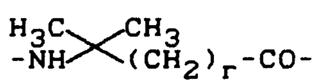

stehen,

worin

r die Zahl 0 oder 1 bedeutet

- für eine Gruppe der Formel

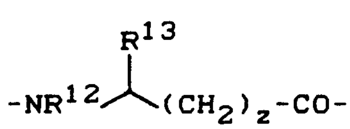

stehen

worin

z - die Zahl 0 oder 1 bedeutet,

$R^{12}$ - Wasserstoff, Methyl oder Ethyl bedeutet,

$R^{13}$

- Cyclopentyl, Cyclohexyl, Phenyl oder Wasserstoff bedeutet,
- oder geradkettiges oder verzweigtes Alkyl mit bis zu 6 Kohlenstoffatomen bedeutet, das gegebenenfalls durch Methylthio, Hydroxy, Mercapto, Guanidyl, Amino, Carboxy oder $H_2N$-CO-substituiert sein kann, oder durch Cyclohexyl,

Naphthyl oder Phenyl substituiert ist, das seinerseits durch Fluor, Chlor, Hydroxy, Nitro oder Alkoxy mit bis zu 4 Kohlenstoffatomen substituiert sein kann, oder durch Indolyl, Imidazolyl, Pyridyl, Triazolyl oder Pyrazolyl substituiert ist, wobei die entsprechenden -NH-Funktionen gegebenenfalls durch Alkyl mit bis zu 4 Kohlenstoffatomen oder durch eine Aminoschutzgruppe geschützt sind,

$R^1$ - für geradkettiges oder verzweigtes Alkyl oder Alkenyl mit bis zu 8 Kohlenstoffatomen steht, die gegebenenfalls durch Cyclopropyl, Cyclobutyl, Cyclopentyl, Cyclohexyl, Cycloheptyl oder Phenyl substituiert sind, das seinerseits durch Fluor, Chlor, Brom, Nitro, Hydroxy oder Amino substituiert sein kann,

n - für die Zahl 1 oder 2 steht,

$R^2$ und $R^{2'}$ gleich oder verschieden sind und
- für Hydroxy, oder
- für geradkettiges oder verzweigtes Alkoxy mit bis zu 4 Kohlenstoffatomen stehen,

G - für eine Gruppe der Formel -SH oder -OH steht,
und

L - für die $-CH_2$ oder -CO-Gruppe steht

und deren physiologisch unbedenklichen Salze.

Besonders bevorzugt sind Verbindungen der allgemeinen Formel (I),
in welcher

W
- für Wasserstoff, tert.Butyloxycarbonyl (BOC) oder Benzyloxycarbonyl (Z) steht, oder
- für Allyl oder Benzyl steht,
- für eine Gruppe der Formel $R^3$-CO-, $R^5R^4$N-CO- oder $R^6$-$SO_2$ steht,
  worin
  $R^3$
  - Wasserstoff, Trifluormethyl oder geradkettiges oder verzweigtes Alkyl mit bis zu 14 Kohlenstoffatomen bedeutet, das gegebenenfalls bis zu 2-fach durch Phenyl, Naphthyl oder Pyridyl substituiert ist, oder
  - Phenyl oder Naphthyl bedeutet, das gegebenenfalls durch Fluor, Chlor, Trifluormethyl, Trifluormethoxy oder durch geradkettiges oder verzweigtes Alkyl mit bis zu 4 Kohlenstoffatomen substituiert ist,
  - Cyclopropyl, Cyclopentyl, Cyclohexyl, Chinolyl-N-oxid, Chinolyl, Indolyl, Pyridyl, Pyridyl-N-oxid, Morpholino oder Piperazinyl bedeutet, oder
  - einen Rest der Formel

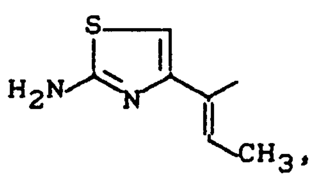,
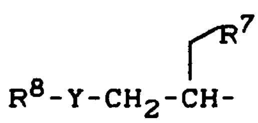
oder
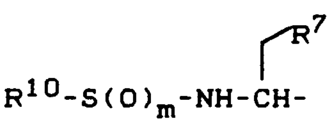

bedeutet,
worin
Y - die CO- oder $SO_2$-Gruppe bedeutet,
$R^7$ - Phenyl oder Naphthyl bedeutet,
$R^8$ und $R^{10}$ gleich oder verschieden sind und geradkettiges oder verzweigtes Alkyl mit bis zu 4 Kohlenstoffatomen, Tolyl, Phenyl oder Naphthyl bedeuten,
m - eine Zahl 1 oder 2 bedeutet,

$R^4$ und $R^5$ gleich oder verschieden sind und
- Wasserstoff oder

- Phenyl oder Naphthyl bedeuten, das gegebenenfalls durch Methyl, Fluor oder Chlor substituiert ist,
- Cyclopropyl, Cyclopentyl oder Cyclohexyl bedeuten, oder
- geradkettiges oder verzweigtes Alkyl mit bis zu 14 Kohlenstoffatomen bedeuten,

$R^6$ - geradkettiges oder verzweigtes Alkyl mit bis 4 Kohlenstoffatomen bedeutet,

A, B und D gleich oder verschieden sind und

- für eine direkte Bindung stehen, oder
- für Prolin stehen, oder
- für einen Rest der Formel

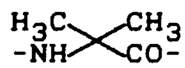

stehen,

- für eine Gruppe der Formel

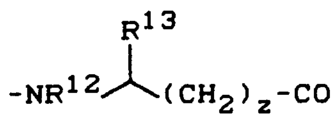

stehen,

worin

z - die Zahl 0 oder 1 bedeutet,

$R^{12}$ - Wasserstoff oder Methyl bedeutet,

$R^{13}$

- Cyclopentyl, Cyclohexyl oder Wasserstoff bedeutet,
- oder geradkettiges oder verzweigtes Alkyl mit bis zu 4 Kohlenstoffatomen bedeutet, das gegebenenfalls durch Methylthio, Hydroxy, Mercapto, Guanidyl, Amino, Carboxy oder $H_2N$-CO- substituiert ist,
  oder durch Cyclohexyl, Naphthyl oder Phenyl substituiert ist, das seinerseits durch Fluor, Chlor oder Alkoxy mit bis zu 4 Kohlenstoffatomen substituiert sein kann, oder
  durch Indolyl, Imidazolyl, Triazolyl, Pyridyl oder Pyrazolyl substituiert ist, wobei die NH-Funktion gegebenenfalls durch Methyl, Benzyloxymethylen oder t-Butyloxycarbonyl (Boc) geschützt ist,

$R^1$ - für geradkettiges oder verzweigtes Alkyl mit bis zu 6 Kohlenstoffatomen steht, das gegebenenfalls durch Cyclopropyl, Cyclopentyl, Cyclohexyl oder Phenyl substituiert ist, das seinerseits durch Hydroxy substituiert sein kann,

n - für die Zahl 1 oder 2 steht,

$R^2$ und $R^{2'}$ gleich oder verschieden sind und

- für Hydroxy oder geradkettiges oder verzweigtes Alkoxy mit bis zu 4 Kohlenstoffatomen stehen,

G - für eine Gruppe der Formel -SH oder -OH steht,

und

L - für die $-CH_2$ oder -CO-Gruppe steht

und deren physiologisch unbedenklichen Salze.

Außerdem wurde ein Verfahren zur Herstellung der erfindungsgemäßen Verbindungen der allgemeinen Formel (I),

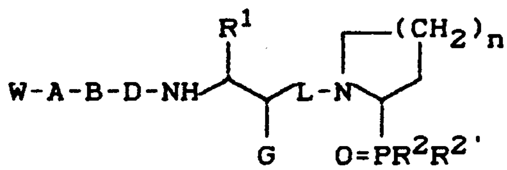

(I)

in welcher
W, A, B, D, $R^1$, $R^2$, $R^{2'}$, G, L und n die oben angegebene Bedeutung haben,
gefunden, dadurch gekennzeichnet, daß man

[A] Verbindungen der allgemeinen Formel (Ia) oder (Ib)

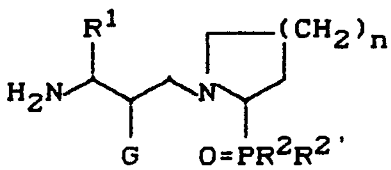

(Ia)

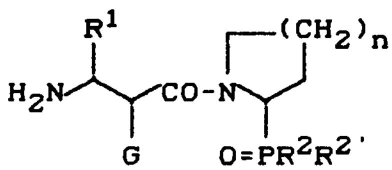

(Ib)

in welcher
$R^1$, $R^2$, $R^{2'}$, G und n die oben angegebene Bedeutung haben,
über die entsprechenden Salze, vorzugsweise über die Hydrochloride,
entweder mit Verbindungen der allgemeinen Formel (II)

W-A'-B'-D'-OH (II)

in welcher
W die oben angegebene Bedeutung hat,
und
A', B' und D' jeweils die oben angegebene Bedeutung von A, B und D haben aber nicht gleichzeitig für eine Bindung stehen,
unter Aktivierung der Carbonsäure, gegebenenfalls in Anwesenheit einer Base und eines Hilfsstoffes, in einem Schritt oder sukzessive (je nach Bedeutung der Substituenten A', B' und D'),
oder mit Verbindungen der allgemeinen Formeln (III) oder (IV)

W-X (III)

$(W')_2O$ (IV)

in welcher
W die oben angegebene Bedeutung hat
X für Halogen, vorzugsweise für Chlor steht,
und
W' für die Gruppe $CF_3CO$- oder $CH_3CO$- steht,
nach den in der Peptidchemie üblichen Bedingungen, in inerten Lösungsmitteln, in Anwesenheit einer Base kondensiert,
oder

[B] im Fall, daß L für die -$CH_2$-Gruppe steht, Verbindungen der allgemeinen Formeln (V) oder (VI)

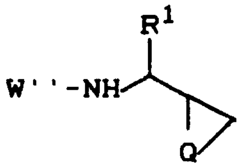

(V)

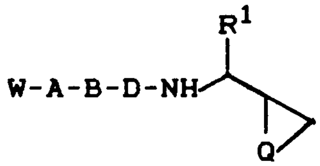

(VI)

in welcher
$R^1$, W, A, B und D die oben angegebene Bedeutung haben
Q für Sauerstoff oder Schwefel steht,
und
W'' für eine Aminoschutzgruppe, vorzugsweise für BOC steht,
mit Verbindungen der allgemeinen Formel (VII)

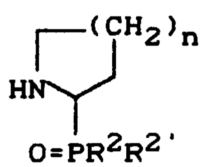

(VII)

in welcher
$R^2$, $R^{2'}$ und n die oben angegebene Bedeutung haben,
in inerten Lösungsmitteln, gegebenenfalls unter Druck umsetzt und im Fall der Verbindungen der allgemeinen Formel (V) die Schutzgruppe W'' anschließend nach üblicher Methode abspaltet und gegebenenfalls mit den Verbindungen der allgemeinen Formel (II) nach der unter Verfahren [A] beschriebenen Methode weiter umsetzt,
oder
[C] im Fall, daß L für die CO-Gruppe steht,
Verbindungen der allgemeinen Formel (VIII)

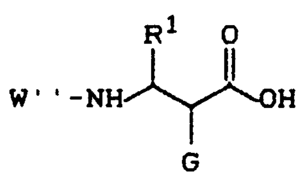

(VIII)

in welcher
W'', G und $R^1$ die oben angegebene Bedeutung haben,
mit den Verbindungen der allgemeinen Formel (VII) unter Aktivierung der Carbonsäure in Anwesenheit einer Base und eines Hilfsstoffes umsetzt und gegebenenfalls unter Abspaltung der Schutzgruppe W'' die Gruppe W-A'-B'-D'-OH nach der unter [A] angegebenen Methode einführt.

Die erfindungsgemäßen Verfahren können durch folgendes Formelschema beispielhaft erläutert werden:

[A]

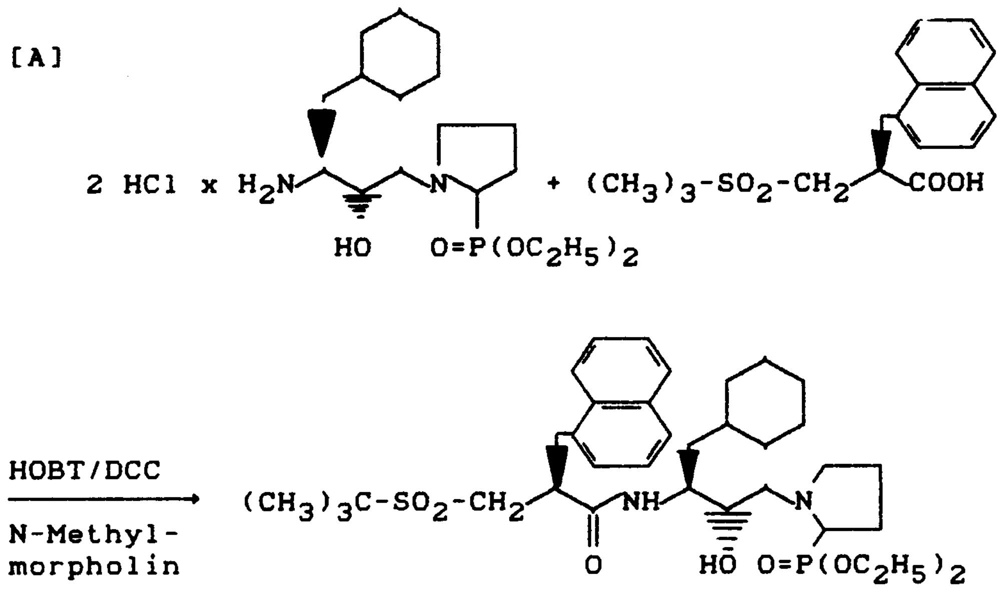
HOBT/DCC
N-Methyl-
morpholin

[B]

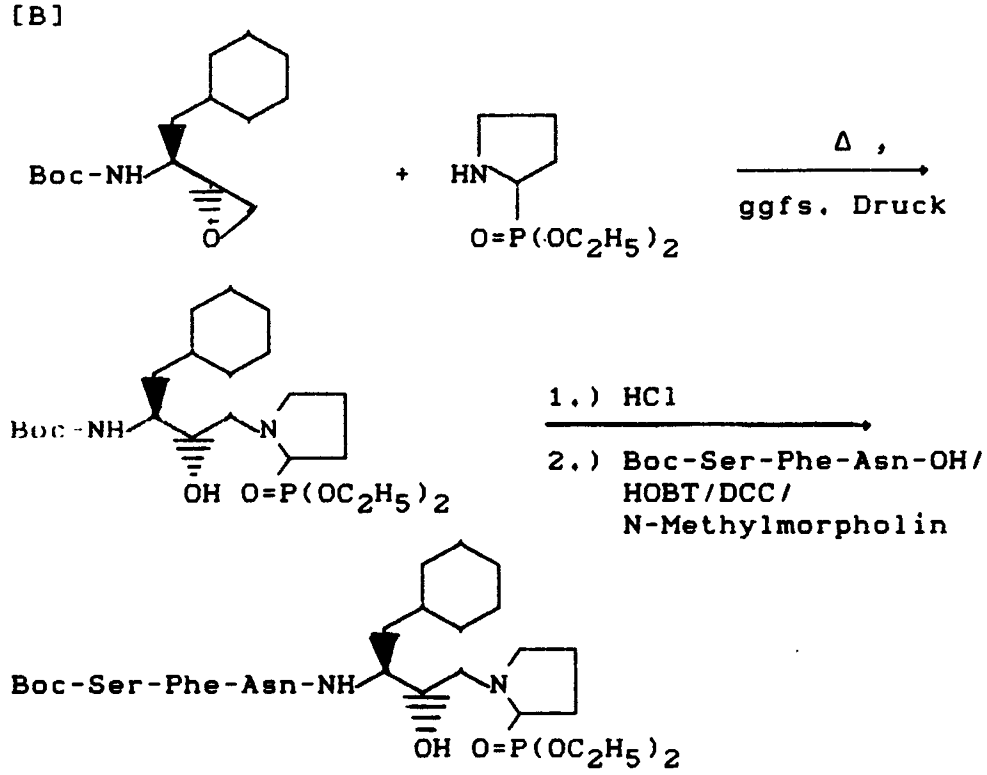
Δ ,
ggfs. Druck
1.) HCl
2.) Boc-Ser-Phe-Asn-OH/
HOBT/DCC/
N-Methylmorpholin

[C]

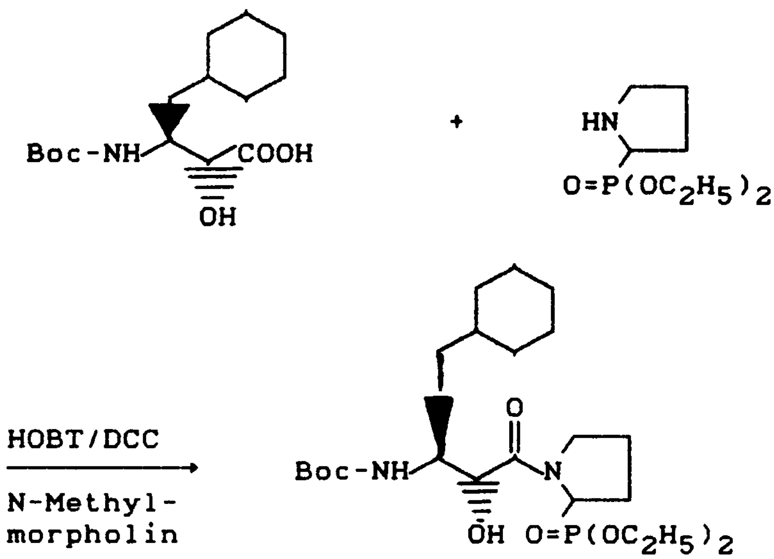


Als Lösungsmittel eignen sich für alle Verfahrensschritte die üblichen inerten Lösungsmittel, die sich unter den Reaktionsbedingungen nicht verändern. Hierzu gehören bevorzugt organische Lösungsmittel wie Ether z.B. Diethylether, Glykolmono-oder dimethylether, Dioxan oder Tetrahydrofuran, oder Kohlenwasserstoffe wie Benzol, Toluol, Xylol, Cyclohexan oder Erdölfraktionen oder Halogenkohlenwasserstoffe wie Methylenchlorid, Chloroform, Tetrachlorkohlenwasserstoff, oder Dimethylsulfoxid, Dimethylformamid, Hexamethylphosphorsäuretriamid, Essigester, Pyridin, Triethylamin oder Picoline. Ebenso ist es möglich, Gemische der genannten Lösungsmittel zu verwenden. Besonders bevorzugt sind Dichlormethan, Chloroform, Dimethylformamid oder Tetrahydrofuran.

Die Verbindungen der allgemeinen Formel (II) sind an sich bekannt und können durch Umsetzung eines entsprechenden Bruchstückes, bestehend aus einer oder mehreren Aminosäuregruppierungen, mit einer freien, gegebenenfalls in aktivierter Form vorliegenden Carboxylgruppe mit einem komplementierenden Bruchstück, bestehend aus einer oder mehreren Aminosäuregruppierungen, mit einer Aminogruppe, gegebenenfalls in aktivierter Form, und durch Wiederholung dieses Vorgangs mit entsprechenden Bruchstücken hergestellt werden, anschließend können gegebenenfalls Schutzgruppen abspalten oder gegen andere Schutzgruppen austauscht werden [vgl. Houben-Weyl, Methoden der organischen Chemie, Synthese von Peptiden II, 4. Aufl. Bd. 15/1, 15/2, Georg Thieme Verlag, Stuttgart].

Als Hilfsstoffe für die jeweiligen Peptidkupplungen und für die Einführung des Restes W (III), (IV) und des Phosphonatrings der Formel (VII) werden bevorzugt Kondensationsmittel eingesetzt, die auch Basen sein können, insbesondere wenn die Carboxylgruppe als Anhydrid aktiviert vorliegt. Bevorzugt werden hier die üblichen Kondensationsmittel wie Carbodiimide z.B. N,N'-Diethyl-, N,N'-Dipropyl-, N,N'-Diisopropyl-, N,N'-Dicyclohexylcarbodiimid, N-(3-Dimethylaminoisopropyl)-N'-ethylcarbodiimid-Hydrochlorid, N-Cyclohexyl-N'-(2-morpholinoethyl)-carbodiimid-metho-p-toluolsulfonat, oder Carbonylverbindungen wie Carbonyldiimidazol, oder 1,2-Oxazoliumverbindungen wie 2-Ethyl-5-phenyl-1,2-oxazolium-3-sulfat oder 2-tert-Butyl-5-methyl-isoxazoliumperchlorat, oder Acylaminoverbindungen wie 2-Ethoxy-1-ethoxycarbonyl-1,2-dihydrochinolin, oder Propanphosphonsäureanhydrid, oder Isobutylchloroformat, oder Benzotriazolyloxy-tris-(dimethylamino)phosphonium-hexafluorophosphat oder 1-Hydroxybenzotriazol.

Außerdem können beispielsweise Alkalicarbonate z.B. Natrium- oder Kaliumcarbonat oder -hydrogencarbonat, oder organische Basen wie Trialkylamine z.B. Triethylamin, N-Ethylmorpholin, N-Methylpiperidin oder N-Methylmorpholin eingesetzt werden. Bevorzugt ist N-Methylmorpholin.

Die Hilfsstoffe und Basen werden in einer Menge von 1,0 Mol bis 3,0 Mol, bevorzugt 1,0 bis 1,2 Mol, bezogen auf jeweils 1 Mol der Verbindungen der allgemeinen Formeln (II), (III), (IV), (VII) und (VIII) eingesetzt.

Die Reaktionen werden in einem Temperaturbereich von 0° C bis 100° C, vorzugsweise bei 0° C bis 30° C und bei Normaldruck durchgeführt.

Die Reaktionen können sowohl bei Normaldruck als auch bei erhöhtem oder erniedrigtem Druck (beispielsweise 0,5 bis 5 bar), vorzugsweise im Fall [A] und [C] bei Normaldruck und im Fall [B] unter

erhöhtem Druck durchgeführt werden.

Die Verbindungen der allgemeinen Formel (Ia) sind neu und können nach dem oben aufgeführten Verfahren [B] hergestellt werden.

Die Verbindungen der allgemeinen Formel (Ib) sind neu und können nach dem oben aufgeführten Verfahren [C] hergestellt werden.

Die Verbindungen der allgemeinen Formeln (III) und (IV) sind bekannt oder können nach üblicher Methode hergestellt werden.

Die Verbindungen der allgemeinen Formel (V) und (VI) sind teilweise bekannt [vgl. J.O.C. 52, 1487 (1987); J. Med. Chem. 31, 1839 (1988)].

Die Verbindungen der allgemeinen Formel (VI), in denen W, $R^1$, Q, A, B und D die oben angegebene Bedeutung haben, aber D nicht für eine direkte Bindung steht, und deren physiologisch unbedenklichen Salze sind neu, ebenfalls pharmakologische Wirkstoffe und werden im folgenden unter der allgemeinen Formel (VIa) erfaßt.

Auch erfindungsgemäßen Verbindungen der allgemeinen Formel (VIa) haben mehrere asymmetrische Kohlenstoffatome. Sie können unabhängig voneinander in der D- oder L-Form vorliegen. Die Erfindung umfaßt die optischen Antipoden ebenso wie die Isomerengemische oder Racemate. Bevorzugt liegen die Gruppen A, B und D unabhängig voneinander in der optisch reinen, bevorzugt in der L-Form vor.

Der Rest der allgemeinen Formel (XIII)

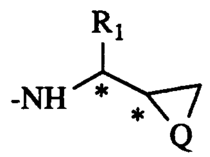

(XIII)

besitzt 2 asymmetrische Kohlenstoffatome (*), die unabhängig voneinander in der R- oder S-Konfiguration vorliegen können.

Die Verbindungen der allgemeinen Formel (VIa)

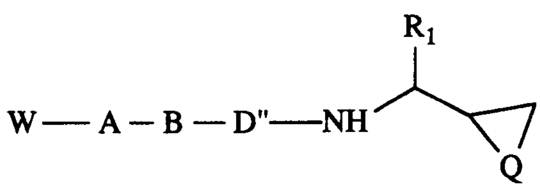

(VIa)

in welcher

W, A, B, $R^1$ und Q die oben angegebene Bedeutung haben

und

D'' die oben angegebene Bedeutung von D hat, aber nicht für eine direkte Bindung steht, können hergestellt werden, indem man

[D] im Fall, daß Q für ein Sauerstoffatom steht, Verbindungen der Formel (IX)

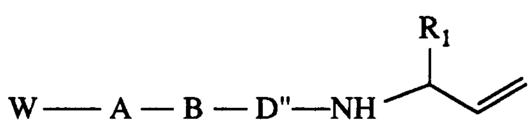

(IX)

in welcher

W, A, B, D'' und $R^1$ die oben angegebene Bedeutung haben,

in inerten Lösemitteln mit einer Epoxidierungsreaktion, gegebenenfalls mit Hilfe einer Base oder einer Phasentransferkatalyse zu Verbindungen der allgemeinen Formel (VIb)

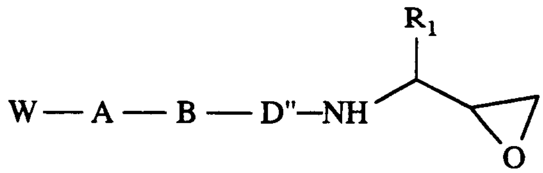
(VIb)

in welcher
W, A, B, D'' und $R^1$ die oben angegebene Bedeutung haben,
umsetzt
und
[E] im Fall, daß Q für ein Schwefelatom steht, die Verbindungen der allgemeinen Formel (VIb) mit Thiodimethylformamid der Formel (X)

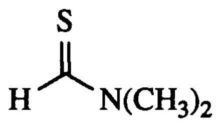
(X)

in inerten Lösemitteln, in Anwesenheit von Säuren, in einer Umlagerungsreaktion zu Verbindungen der allgemeinen Formel (VIc)

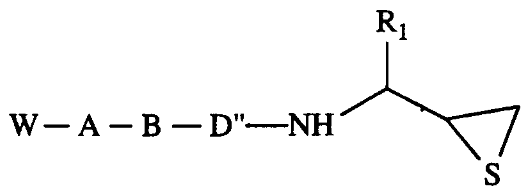
(VIc)

in welcher
W, A, B, D'' und $R^1$ die oben angegebene Bedeutung haben,
weiter umsetzt.

Das erfindungsgemäße Verfahren kann durch folgendes Formelschema beispielhaft erläutert werden:

[D]

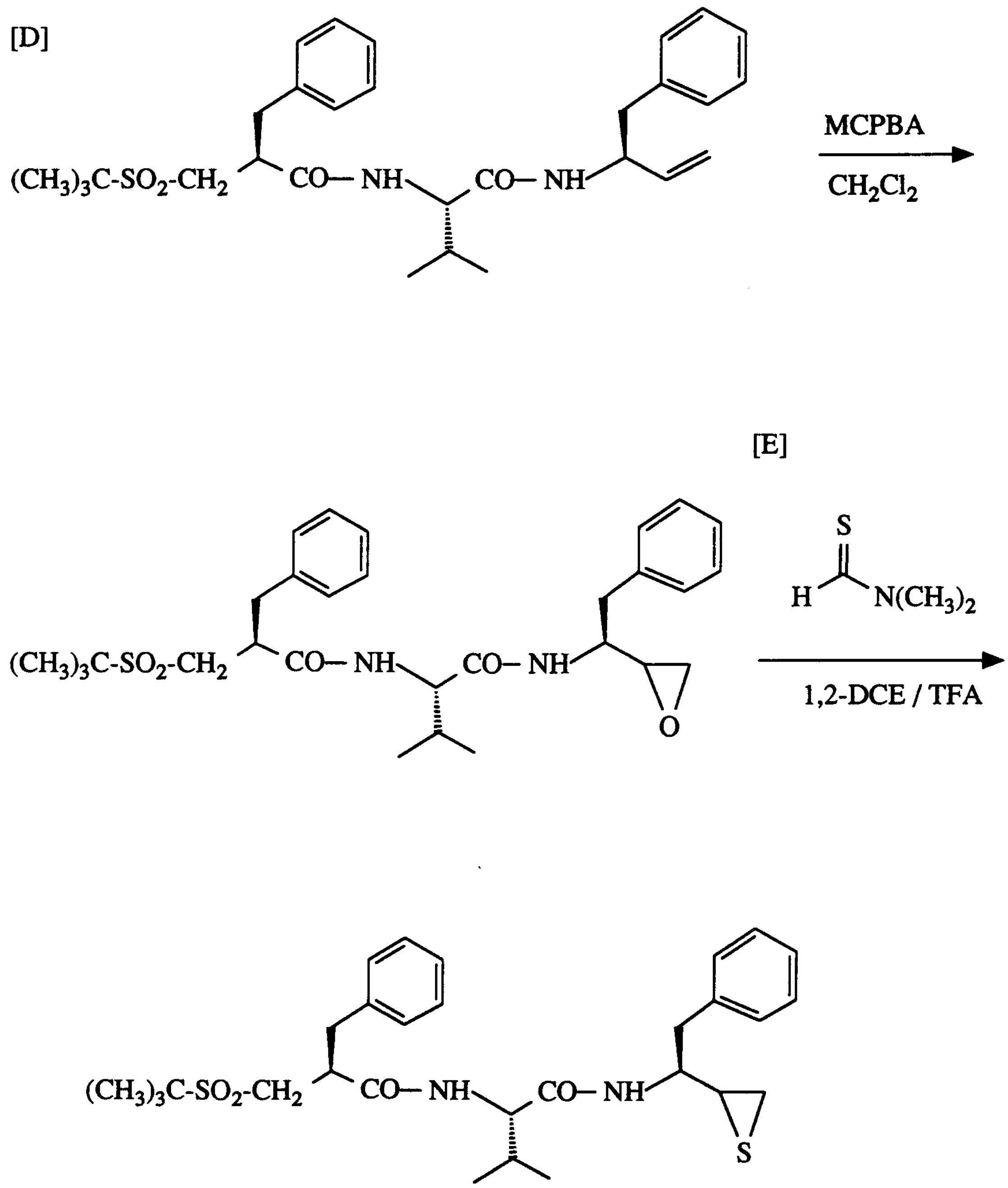


[E]

Als Lösemittel für die Verfahren [D] und [E] eignen sich die üblichen inerten Lösemittel, die sich unter den Reaktionsbedingungen nicht verändern. Hierzu gehören bevorzugt organische Lösemittel wie Ether z.B. Diethylether, Glykolmono- oder -dimethylether, Dioxan oder Tetrahydrofuran, oder Kohlenwasserstoffe wie Benzol, Toluol, Xylol, Cyclohexan oder Erdölfraktionen oder Halogenkohlenwasserstoffe wie Methylenchlorid, Dichlorethan (DCE), Chloroform, Tetrachlorkohlenstoff, oder Dimethylsulfoxid, Dimethylformamid, Hexamethylphosphorsäuretriamid, Essigester, Pyridin, Triethylamin oder Picolin. Ebenso ist es möglich, Gemische der genannten Lösemittel zu verwenden. Besonders bevorzugt sind Dichlormethan, Dichlorethan oder Chloroform.

Als Reagentien für die Epoxidierung eignen sich die literaturbekannten Verbindungen wie beispielsweise m-Chlorbenzoesäure, Magnesiummonoperoxyphthalat, Dimethyldioxiran oder Methyl(trifluormethyl)dioxiran. Bevorzugt sind m-Chlorperbenzoesäure und Magnesiummonoperoxyphthalat [vgl. P. Brongham et al., Synthesis (1987), 1015; W. Adam et al., J. Org. Chem. 52, 2800 (1987) und R. Curci et al., J. Org. Chem. 53, 3890 (1988)].

Wird die Epoxidierung mit Hilfe einer Phasentransfer-Katalyse durchgeführt so werden als Hilfsstoffe beispielsweise organische Ammoniumchloride oder -bromide wie beispielsweise Benzyltriethylammoniumchlorid oder -bromid, Methyltrioctylammoniumchlorid, Tetrabutylammoniumbromid, Tricaprylmethylammoniumchlorid (Aliquat 336) eingesetzt. Bevorzugt sind Benzyltriethylammoniumchlorid und -bromid.

Die Umlagerung erfolgt in Analogie zu einer literaturbekannten Methode [vgl. T. Takido et al., Synthesis (1986), 779].

Als Säuren eignen sich bei der Umlagerung beispielsweise Methansulfonsäure, Trifluoressigsäure, Trifluormethansulfonsäure oder Tetrafluoroborstoffsäure. Bevorzugt ist Trifluoressigsäure.

Die Säure wird in einer Menge von 0,01 mol bis 0,1 mol, bevorzugt in katalytischen Mengen, bezogen auf 1 mol der Verbindung der Formel (X) eingesetzt.

Die Epoxidierung und die Umlagerung werden in einem Temperaturbereich von -10°C bis +90°C, bevorzugt von 0°C bis +60°C durchgeführt.

Die Reaktionen können sowohl bei Normaldruck als auch bei erhöhtem oder erniedrigtem Druck (beispielsweise 0,5 bis 5 bar), bevorzugt bei Normaldruck durchgeführt werden.

Die Verbindungen der allgemeinen Formel (IX) sind neu und können hergestellt werden, indem man

[F] Verbindungen der allgemeinen Formel (XI)

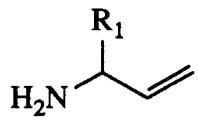

(XI)

in welcher

$R^1$ die oben angegebene Bedeutung hat,

mit Verbindungen der allgemeinen Formel (IIa)

W-A-B-D''-OH (IIa)

in welcher

W, A, B und D'' die oben angegebene Bedeutung haben,

unter Aktivierung der Carbonsäure, gegebenenfalls in Anwesenheit einer Base und eines Hilfsstoffes, in einem Schritt oder sukzessive (je nach Bedeutung der Substituenten A, B und D'') umsetzt, oder

[G] Verbindungen der allgemeinen Formel (IXa)

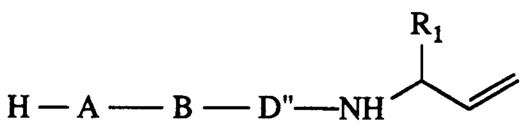

(IXa)

in welcher

A, B, D'' und $R^1$ die oben angegebene Bedeutung haben,

mit den oben aufgeführten Verbindungen der allgemeinen Formeln (III) oder (IV)

W-X (III)

$(W')_2O$ (IV)

in welchen

W, X und W' die oben angegebene Bedeutung haben

nach den in der Peptidchemie üblichen Bedingungen in inerten Lösemitteln, in Anwesenheit einer Base kondensiert.

Hinsichtlich der Wahl der Lösungsmittel, Basen und Hilfsstoffe für die Verfahren [G] und [F] gelten die unter Verfahren [A] oben aufgeführten Kriterien.

Die Reaktionen [F] und [G] werden in einem Temperaturbereich von 0°C bis 100°C, vorzugsweise bei 0°C bis 30°C und bei Normaldruck durchgeführt.

Die Reaktionen [F] und [G] können sowohl bei Normaldruck als auch bei erhöhtem oder erniedrigtem

Druck (beispielsweise 0,5 bis 5 bar), vorzugsweise bei Normaldruck durchgeführt werden.

Die Verbindungen der allgemeinen Formel (IXa) sind neu und können nach dem oben aufgeführten Verfahren [G] hergestellt werden.

Die Verbindungen der allgemeinen Formel (XI) sind an sich bekannt oder können nach literaturbekannter Methode hergestellt werden [vgl. J.R. Luly et al., J. Org. Chem. 52, (1987), 1487].

Die Verbindungen der allgemeinen Formel (VII) sind ebenfalls bekannt [n=1 vgl. US 4 186 268; Y. Nomura et al., Chem. Lett., 693(1977); n = 2 vgl. V.A. Solodenko et al., Zh. Obshch. Khim. 57, 2392 (1987)-].

Die Verbindungen der allgemeinen Formel (VIII) sind bekannt oder können nach üblichen Methoden hergestellt werden [vgl. DOS 3 825 242; EP 252 727; EP 244 084; US 4 599 198 und EP 266 950].

Es wurde überraschend gefunden, daß die Verbindungen der allgemeinen Formeln (I) und (VIa) eine außerordentlich starke Wirkung gegen Retroviren besitzen. Dies wird mit einem HIV-spezifischen Protease-Enzymtest belegt.

Die Ergebnisse der unten aufgeführten Beispiele wurden nach dem in den folgenden Literaturangaben [vgl. Hansen, J., Billich, S., Schulze, T., Sukrow, S. and Mölling, K. (1988), EMBO Journal, Vol, 7, No. 6, pp. 1785-1791] beschriebenen HIV-Testsystem ermittelt: Gereinigte HIV-Protease wurde mit synthetischem Peptid, das eine Schnittstelle im Gag-Precursor-Protein imitiert und eine in vivo-Spaltstelle der HIV-Protease darstellt, inkubiert. Die entstandenen Spaltprodukte des synthetischen Peptids wurden über Reverse Phase High Performance Liquid Chromatography (RP-HPLC) analysiert. Die angegebenen $IC_{50}$-Werte beziehen sich auf die Substanzkonzentration, die unter den oben aufgeführten Testbedingungen eine 50 %ige Hemmung der Protease-Aktivität bewirkt.

Verbindungen der allgemeinen Formel (I)

| Bsp.-Nr. | $IC_{50}$ (RP-HPLC) (M) | Bsp. -Nr. | $IC_{50}$ (RP-HPLC) (M) |
|---|---|---|---|
| 5 (unpolar) | $10^{-6}$ | 26 (polar) | $10^{-7}$ |
| 5 (polar) | $10^{-7}$ | 27 (polar) | $< 10^{-8}$ |
| 10 | $5 \times 10^{-8}$ | 28 (unpolar) | $10^{-6}$ |
| 11 | $5 \times 10^{-7}$ | 29 (polar) | $10^{-9}$ |
| 17 | $10^{-8}$ | 30 (polar) | $10^{-7}$ |
| 18 | $10^{-9}$ | 38 (polar) | $5 \times 10^{-7}$ |
| 22 | $10^{-7}$ | 40 (polar) | $5 \times 10^{-7}$ |
| 25 | $10^{-6}$ | | |

Verbindungen der allgemeinen Formel (VIa)

$IC_{50}$(RP-HPLC)(M)

| Bsp.-Nr. | HIV-1 | HIV-2 |
|---|---|---|
| 46 | $5 \times 10^{-9}$ | $10^{-8}$ |
| 48 | $10^{-10}$ | not tested |
| 49 | $10^{-9}$ | not tested |
| 50 | $10^{-9}$ | not tested |
| 52 | $10^{-8}$ | $10^{-9}$ |
| 54 | $10^{-8}$ | $10^{-8}$ |
| 55 | $10^{-8}$ | $5 \times 10^{-8}$ |
| 56 | $10^{-8}$ | not tested |
| 57 | $10^{-7}$ | not tested |
| 59 | $10^{-8}$ | $10^{-9}$ |
| 60 | $10^{-9}$ | $5 \times 10^{-8}$ |
| 61 | $5 \times 10^{-8}$ | not tested |
| 62 | $10^{-8}$ | $5 \times 10^{-7}$ |
| 63 | $10^{-8}$ | $10^{-6}$ |
| 64 | $5 \times 10^{-7}$ | not tested |
| 65 | $10^{-6}$ | " |
| 66 | $10^{-7}$ | " |
| 78 | $10^{-7}$ | " |

Außerdem zeigten die erfindungsgemäßen Verbindungen Wirkung in Lentivirus infizierten Zellkulturen. Dies konnte am Beispiel des HIV-Virus gezeigt werden.

HIV-Infektion in Zellkultur:

Der HIV-Test wurde mit geringen Modifikationen nach der Methode von Pauwels et al. (Journal of Virological Methods 20 (1988) 309-321) durchgeführt.

Normale menschliche Blutlymphozyten (PBL's) wurden über Ficoll-Hypaque angereichert und in RPMI 1640, 20 % fötales Kälberserum mit Phythaemagglutinin (90 µg/ml) und Interleukin-2 (40 U/m) stimuliert. Zur Infektion mit dem infektiösen HIV wurden PBL's pelletiert und das Zellpellet wurde anschließend in 1 ml HIV-Virusadsorptionslösung suspendiert und 1 Std. bei 37° C inkubiert.

Die Virusadsorptionslösung wurde zentrifugiert und das infizierte Zellpellet in Wachstumsmedium aufgenommen, so daß $1 \times 10^5$ Zellen pro ml eingestellt waren. Die derart infizierten Zellen wurden zu $1 \times 10^4$ Zellen/Napf in die Näpfe von 96er Mikrotiterplatten pipettiert.

Die erste vertikale Reihe der Mikrotiterplatte enthielt nur Wachstumsmedium und Zellen, die nicht infiziert, aber ansonsten genauso wie oben beschrieben, behandelt worden waren (Zellkontrolle). Die zweite vertikale Reihe der Mikrotiterplatte erhielt nur HIV-infizierte Zellen (Viruskontrolle) in Wachstumsmedium. Die übrigen Näpfe enthielten die erfindungsgemäßen Verbindungen in unterschiedlichen Konzentrationen, ausgehend von den Näpfen der 3. vertikalen Reihe der Mikrotiterplatte, von der die Prüfverbindungen in 2er Schritten 10fach verdünnt wurden.

Die Testansätze wurden so lange bei 37° C inkubiert, bis in der unbehandelten Viruskontrolle die für das HIV typische Syncytienbildung auftrat (zwischen Tag 3 und 6 nach Infektion), die dann mikroskopisch ausgewertet wurde. In der unbehandelten Viruskontrolle resultierten unter diesen Testbedingungen etwa 20 Syncytien, während die unbehandelte Zellkontrolle keine Syncytien aufwies.

Die IC-50 Werte wurden als die Konzentration der behandelten und infizierten Zellen ermittelt, bei der 50 % (ca. 10 Syncytien) der virusinduzierten Syncytien durch die Behandlung mit der erfindungsgemäßen Verbindung unterdrückt waren.

| Beispiel-Nr.: | IC-50 ($\mu$M) |
|---|---|
| allg. Formel (I) 27 | 9,5 |
| allg. Formel (I) 29 | 4,7 |
| allg. Formel (I) 32 | 5,7 |
| allg. Formel (VIa) 49 | 10 |
| allg. Formel (VIa) 49 | 10 |

Es wurde gefunden, daß die erfindungsgemäßen Verbindungen HIV infizierte Zellen vor der virusinduzierten Zellzerstörung schützen.

Die erfindungsgemäßen Verbindungen stellen wertvolle Wirkstoffe zur Behandlung und Prophylaxe von Erkrankungen, hervorgerufen durch Retroviren, in der Human- und Tiermedizin dar.

Als Indikationsgebiete in der Humanmedizin können beispielsweise genannt wurden:

1.) Die Behandlung oder Prophylaxe von menschlichen Retrovirusinfektionen.

2.) Für die Behandlung oder Prophylaxe von HIV I (Virus der humanen Immundefizienz; früher HTLV III/LAV genannt) und HIV II verursachten Erkrankungen (AIDS) und den damit assozierten Stadien wie ARC (AIDS related complex) und LAS (Lymphadenopathie-Syndrom) sowie der durch dieses Virus verursachten Immunschwäche und Encephalopathie.

3.) Für die Behandlung oder die Prophylaxe einer HTLV I-oder HTLV II-Infektion.

4.) Für die Behandlung oder die Prophylaxe des AIDS-carrier Zustandes (AIDS-Überträger-Zustand).

Als Indikationen in der Tiermedizin können beispielsweise angeführt werden:

Infektionen mit

a) Maedivisna (bei Schafen und Ziegen)
b) progressivem Pneumonievirus (PPV) (bei Schafen und Ziegen)
c) caprine arthritis encephalitis Virus (bei Schafen und Ziegen)
d) Zwoegerziekte Virus (bei Schafen)
e) infektiösem Virus der Anämie (des Pferdes)
f) Infektionen verursacht durch das Katzenleukämievirus
g) Infektionen verursacht durch das Virus der Katzen-Immundefizienz
h) Infektionen verursacht durch den Simian-Immunschwäche-Virus

Bevorzugt werden aus dem Indikationsgebiet in der Humanmedizin die oben aufgeführten Punkte 2, 3 und 4.

Zur vorliegenden Erfindung gehören pharmazeutische Zubereitungen, die neben nicht-toxischen, inerten pharmazeutisch geeigneten Trägerstoffen eine oder mehrere Verbindungen der Formeln (I) und (VIa) enthalten oder die aus einem oder mehreren Wirkstoffen der Formel (I) oder (VIa) bestehen, sowie Verfahren zur Herstellung dieser Zubereitungen.

Die Wirkstoffe der Formeln (I) und (VIa) sollen in den oben aufgeführten pharmazeutischen Zubereitungen, vorzugsweise in einer Konzentration von etwa 0,1 bis 99,5, vorzugsweise von etwa 0,5 bis 95 Gew.-%, der Gesamtmischung vorhanden sein.

Die oben aufgeführten pharmazeutischen Zubereitungen können außer den Verbindungen der Formeln (I) und (VIa) auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Im allgemeinen hat es sich sowohl in der Human- als auch in der Veterinärmedizin als vorteilhaft erwiesen, den oder die Wirkstoffe der Formeln (I) und (VIa) in Gesamtmengen von etwa 0,5 bis etwa 500, vorzugsweise 5 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die Wirkstoffe vorzugsweise in Mengen von etwa 1 bis etwa 80, insbesondere 1 bis 30 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt.

Anhang zum experimentellen Teil

I. Liste der verwendeten Laufmittelgemische zur Chromatographie:

I Dichlormethan : Methanol
II Toluol : Ethylacetat
III Acetonitril : Wasser
IV Dichlormethan : Methanol : Ammoniak 9:1:0,1

## II. Aminosäuren

Im allgemeinen erfolgt die Bezeichnung der Konfiguration durch das Vorausstellen eines L bzw. D vor der Aminosäureabkürzung, im Fall des Racemats durch ein D,L- wobei zur Vereinfachung bei L-Aminosäuren die Konfigurationsbezeichnung unterbleiben kann und dann nur im Fall der D-Form bzw. des D,L-Gemisches explizierte Bezeichnung erfolgt.

| | |
|---|---|
| Ala | L-Alanin |
| Arg | L-Arginin |
| Asn | L-Asparagin |
| Asp | L-Asparaginsäure |
| Cys | L-Cystein |
| Gln | L-Glutamin |
| Glu | L-Glutaminsäure |
| Gly | L-Glycin |
| His | L-Histidin |
| Ile | L-Isoleucin |
| Leu | L-Leucin |
| Lys | L-Lysin |
| Met | L-Methionin |
| Pro | L- Prolin |
| Phe | L-Phenylalanin |
| Ser | L-Serin |
| Thr | L-Threonin |
| Trp | L-Tryptophan |
| Tyr | L-Tyrosin |
| Val | L-Valin |

## III. Abkürzungen

| | |
|---|---|
| Z | Benzyloxycarbonyl |
| BOC | tert.Butoxycarbonyl |
| CMCT | 1-Cyclohexyl-3-(2-morpholino-ethyl)-carbodiimid-metho-p-toluolsulfonat |
| DCC | Dicyclohexylcarbodiimid |
| DMF | Dimethylformamid |
| HOBT | 1-Hydroxybenzotriazol |
| Mir | Miristoyl |
| Ph | Phenyl |
| THF | Tetrahydrofuran |
| Cha | Cyclohexylalanin |
| MCPBA | m-Chlorperbenzoesäure |
| MMPP | Magnesiummonoperoxyphthalat Hexahydrat |
| Aib | 2-Amino-2-methylpropionsäure |

## Experimenteller Teil I

## Herstellungsbeispiele

### Beispiel 1

1-{(2S, 3S)-3-[(tert.Butoxycarbonyl)amino]-2-hydroxy-4-phenyl-butyl}-(2R,S)-2-(pyrrolidinyl)phosphonsäure-Mononatriumsalz

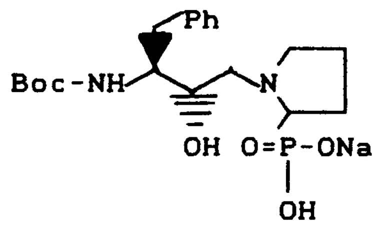

Eine Lösung von 1,22 g (8,08 mmol) 2-Phosphonopyrrolidin [US 4 186 268] in 20 ml Wasser wurde durch Zugabe von 8 ml 1 N NaOH-Lösung auf pH 8 gestellt. Dazu gab man eine Lösung von 2,13 g (8,08 mmol) (1S)-1-[1-(tert.Butoxycarbonyl)amino]-2-phenyl-(1S)-ethyl]oxiran [J.R. Luly et al., J. Org. Chem. 52, 1487 (1987)] in 10 ml Acetonitril und erhitzte das gerührte Gemisch für 2 h bei 105° C in einem Druckgefäß. Nach dem Abkühlen wurde die Reaktionsmischung in 40 ml Wasser gegossen, mit 50 ml Ethylacetat gewaschen, die wässrige Phase im Vakuum auf ein Volumen von ca. 20 ml eingeengt und auf eine Lobar-Fertigsäule/Merck-Lichroprep RP-8 (40-63 µm) Größe C geladen. Es wurde mit Wasser, welches einen steigenden Anteil an Acetonitril enthielt, eluiert. Die produkthaltigen Fraktionen wurden vereinigt und gefriergetrocknet. Man erhielt 1,67 g (47% der Theorie) der Titelverbindung als farbloses Lyophilisat.
$R_f$ = 0,07, III (4:1)
MS (FAB): m/z = 415 $(M+H)^+$, 437 $(M+Na)^+$, 459 $(M+2Na-H)^+$, 481 $(M+3Na-2H)^+$.

Beispiel 2

1-[(2S, 3S)-3-Amino-2-hydroxy-4-phenyl-butyl]-(2R,S)-2-(pyrrolidinyl)phosphonsäure Dihydrochlorid

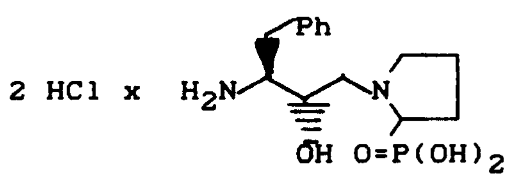

Eine Lösung von 1,41 g (2,61 mmol) der Verbindung aus Beispiel 1 in 13 ml einer 4 N Lösung gasförmigen Chlorwasserstoffs in wasserfreiem Dioxan wurde 30 min bei 0° C gerührt. Danach gab man 10 ml Toluol zu und engte im Vakuum ein. Dieser Vorgang wurde noch zweimal wiederholt, dann wurde der Rückstand mit Ether verrieben, abgesaugt und im Hochvakuum über KOH getrocknet. Man erhielt 1,06 g (99% der Theorie) der Titelverbindung als farbloses Pulver.
$R_f$ = 0,10, III (3:2)
MS (FAB): m/z = 315 $(M+H)^+$, 337 $(M+Na)^+$, 359 $(M+2Na-H)^+$

Beispiel 3

1-{(2S, 3S)-3-[[2-(2-Aminothiazol-4-yl)-2(Z)-butenoyl]-amino]-2-hydroxy-4-phenyl-butyl}-(2R und 2S)-2-(pyrrolidinyl)phosphonsäure-Mononatriumsalz

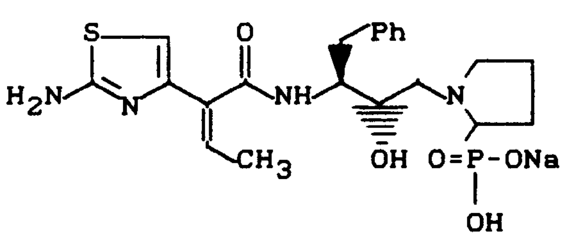

Eine auf 0° C gekühlte, gerührte Lösung von 184 mg (1,0 mmol) 2-(2-Aminothiazol-4-yl)-2(Z)-butensäure [US 4 500 716] in 4 ml wasserfreiem DMF wurde nacheinander mit 149 mg (1,10 mol) HOBT und 206 mg (1,00 mmol) DCC versetzt. Das Kühlbad wurde entfernt und man rührte 1 h bei Raumtemperatur. Danach wurde erneut auf 0° C gekühlt und man tropfte eine Lösung von 375 mg (0,91 mmol) der Verbindung aus Beispiel 2 und 0,32 ml (2,89 mmol) N-Methylmorpholin in 4 ml DMF zu. Das Kühlbad wurde entfernt und man rührte 16 h bei Raumtemperatur nach. Der entstandene Niederschlag wurde durch Filtration abgetrennt, das Filtrat mit 10 ml Toluol versetzt und im Vakuum eingeengt. Der zurückbleibende Schaum wurde in 15 ml Wasser aufgenommen und durch Zugabe von 1 N NaOH pH 7,6 eingestellt. Die Wasserphase wurde zweimal mit 10 ml Ethylacetat gewaschen und dann an einer Lobar-Fertigsäule/Merck-Lichroprep RP-8 (40 -63 µm) Größe B chromatographiert. Es wurde mit Wasser, welches einen steigenden Anteil Acetonitril enthielt, eluiert. Die produkthaltigen Fraktionen wurden vereinigt und gefriergetrocknet. Man erhielt 58 mg (12% der Theorie) des polaren Diastereomers.
$R_f$ = 0,19, III (7:3)
MS (FAB): m/z = 481 $(M+H)^+$, 503 $(M+Na)^+$, 525 $(M+2Na-H)^+$
IR (KBr): 3410, 1644, 1632, 1530, 1088, 978 $cm^{-1}$ und 80 mg (16% der Theorie) des unpolaren Diastereomers
$R_f$ = 0,23, III (7:3)
MS (FAB): m/z = 481 $(M+H)^+$, 503 $(M+Na)^+$, 525 $(M+2Na-H)^+$, 547 $(M-3Na-2H)^+$
IR (KBr): 3200 - 3400, 1656, 1620, 1520, 1088, 978 $cm^{-1}$

## Beispiel 4

1-{-(2S, 3S)-3-[(tert.Butoxycarbonyl)amino]-2-hydroxy-4-phenylbutyl}-(2R und 2S)-2-(pyrrolidinyl)-phosphonsäurediethylester

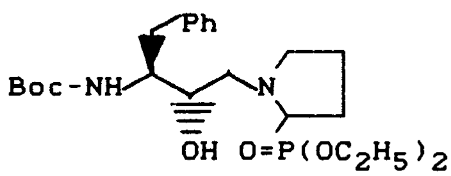

Eine Lösung von 2,63 g (10,0 mmol) (1S)-[1-[1-(tert.-Butoxycarbonyl)-amino]-2-phenyl-(1S)-ethyl]oxiran [J.R. Luly et al., J. Org. Chem. 52, 487 (1987)] und 2,49 g (12,0 mmol) 2-(Pyrrolidinyl)-phosphonsäurediethylester [US 4 186 268] in 5 ml n-Propanol wurde in einem Druckgefäß 2 h bei 110° C gerührt. Nach dem Abkühlen wurde das Reaktionsgemisch im Vakuum eingeengt und nach einer Vorreinigung an 100 g Kieselgel an 400 g Kieselgel (Toluol: Ethylacetat 1:4) chromatographisch aufgetrennt. Man erhielt 1,20 g (26% der Theorie) des unpolaren Diastereomers als Öl.
$R_f$ = 0,32, II (1:4)
MS (FAB): m/z = 471 $(M+H)^+$, 493 $(M+Na)^+$
sowie 1,53 g (32% der Theorie) des polaren Diastereomers als Öl
$R_f$ = 0,24, II (1:4)
MS (FAB): m/z = 471 $(M+H)^+$, 493 $(M+Na)^+$

## Beispiel 5

1-{(2S, 3S)-3-[(tert.Butoxycarbonyl)-amino]-4-cyclohexyl-2-hydroxy-butyl}-(2R und 2S)-2-(pyrrolidinyl)-phosphonsäurediethylester

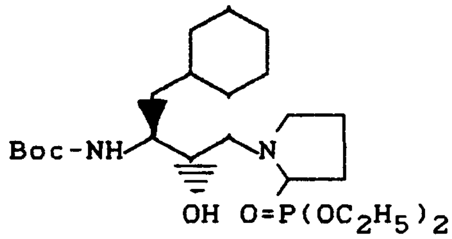

Wie für Beispiel 4 beschrieben erhielt man aus 4,25 g (15,78 mmol) (1S)-1-[1-(tert.Butoxycarbonyl)-amino]-2-cyclohexyl-(1S)-ethyl]oxiran [J.R. Luly et al., J. Org. Chem. 52, 487 (1987)] und 3,92 g (18,94 mmol) 2-(Pyrrolidinyl)phosphonsäurediethylester [US 4 186 268] und Chromatographie des Rohgemisches an 830 g Kieselgel (Toluol : Ethylacetat 1:4) 3,43 g (46% der Theorie) des amorphen unpolaren Diastereomers als Öl

$R_f$ = 0,29, II (1:4)

MS (DCI, $NH_3$): m/z = 477 $(M+H)^+$

und 2,90 g (39% der Theorie) des polaren Diastereomers als Öl

$R_f$ = 0,21, II (1:4)

MS (DCI, $NH_3$): m/z = 477 $(M+H)^+$.

Wie für Beispiel 2 beschrieben, erhielt man aus den entsprechenden Boc-geschützten Verbindungen die folgenden Produkte (Tabelle 1)

Tabelle 1

2 HCl x 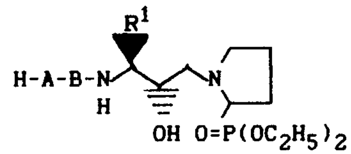

| Beispiel-Nr. | A-B | $R^1$ | Ausbeute (%) | MS (FAB) m/z $(M+H)^+$ | $R_f$/Laufmittel- (Verhältnis) | Startmaterial aus Beispiel |
|---|---|---|---|---|---|---|
| 6 | - | $PhCH_2$ | 70 | 371 | 0,48, I (4:1) | 4 (unpolar) |
| 7 | - | $PhCH_2$ | 96 | 371 | 0,43, I (4:1) | 4 (polar) |
| 8 | Val | $PhCH_2$ | 94 | 470 | 0,12, I (9:1) | 16 (unpolar) |
| 9 | Val | $PhCH_2$ | 79 | 470 | 0,11, I (9:1) | 15 (polar) |
| 10 | Phe-Val | $PhCH_2$ | 72 | 617 | 0,29, I (9:1) | 20 |
| 11 | Phe-Val | $PhCH_2$ | 63 | 617 | 0,36, I (9:1) | 19 |
| 12 | - | 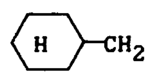 | 69 | 377 | 0,01, II (1:9) | 5 (unpolar) |
| 13 | - | 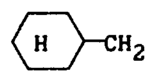 | 87 | 377 | 0,01, II (1:9) | 5 (polar) |

Fortsetzung Tabelle 1

| Beispiel-Nr. | A-B | $R^1$ | Ausbeute (%) | MS (FAB) m/z $(M+H)^+$ | $R_f$/Laufmittel (Verhältnis) | Startmaterial aus Beispiel |
|---|---|---|---|---|---|---|
| 14 | Asn | 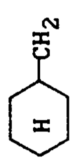 | 85 | 491[a] | 0,40, I (4:1) | 23 |
| 14a | Val | 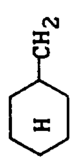 | 88 | 476 | 0,03, I (9:1) | 31 |
| 14b | Val | 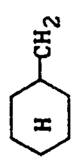 | 96 | 476 | 0,02, I (9:1) | 32 |

a: MS (DCI, $NH_3$): m/z = $(M+H)^+$.

Beispiel 15

1-{(2S, 3S)-3-[(tert.-Butoxycarbonyl)valinylamino]-2-hydroxy-4-phenyl-butyl}-(2R oder 2S)-2-(pyrrolidinyl)-phosphonsäurediethylester

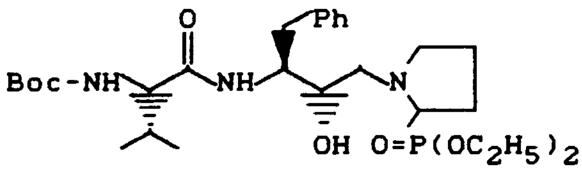

Eine gerührte Lösung von 429 mg (1,97 mmol) N-(tert.-Butoxycarbonyl)-L-Valin in 5 ml wasserfreiem DMF wurde bei 0° C mit 293 mg (2,17 mmol) HOBT und 876 mg (2,07 mmol) CMTC versetzt. Das Kühlbad wurde entfernt und man rührte 1 h bei Raumtemperatur. Danach wurde erneut auf 0° C gekühlt und man gab eine Lösung von 795 mg (1,79 mmol) des polaren Diastereomers aus Beispiel 7 und 0,69 ml (6,27 mmol) N-Methylmorpholin in 4 ml DMF zu. Das Kühlbad wurde entfernt und man rührte 16 h bei Raumtemperatur. Danach wurde im Vakuum eingeengt und der Rückstand zwischen 40 ml Ethylacetat und 40 ml Wasser verteilt. Die Wasserphase wurde mit 10 ml Ethylacetat extrahiert, die vereinigten Extrakte wurden mit 50 ml Wasser gewaschen und über $MgSO_4$ getrocknet. Nach Abdampfen des Lösemittels im Vakuum und Chromatographie des Rohproduktes an 210 g Kieselgel (Dichlormethan : Methanol 95:5) erhielt man 581 mg (57% der Theorie) der Titelverbindung als Öl.

$R_f$ = 0,08, I (95:5), polares Diastereomer

MS (FAB): m/z = 570 $(M+H)^+$, 592 $(M+Na)^+$

Wie für Beispiel 15 beschrieben, erhielt man durch Kupplung der entsprechenden Säuren mit den Aminhydrochloriden (Startmaterial) die folgenden Produkte (Tabelle 2):

Tabelle 2

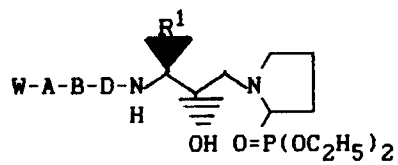

| Beispiel-Nr. | W-A-B-D- | $R^1$ | Ausbeute (%) | MS (FAB) m/z $(M+H)^+$ | $R_f$/Laufmittel- (Verhältnis) | Startmaterial aus Beispiel |
|---|---|---|---|---|---|---|
| 16 | Boc-Val | $PhCH_2$ | 60 | 570[a] | 0,24, I (95:5) | 6 |
| 17 | Boc-Phe-Val | $PhCH_2$ | 74 | 717 | 0,23, I (95:5) | 8 |
| 18 | Boc-Phe-Val | $PhCH_2$ | 69 | 717 | 0,18, I (95:5) | 9 |
| 19 | Boc-Phe-Val | $PhCH_2$ | 48 | 717 | 0,23, I (95.5) | 6 |
| 20 | Boc-Phe-Val | $PhCH_2$ | 42 | 717 | 0,18, I (95:5) | 7 |
| 21 | Boc-Ser-Phe-Val | $PhCH_2$ | 15 | 804 | 0,27, I (93:7) | 11 |
| 22 | Boc-Ser-Phe-Val | $PhCH_2$ | 32 | 804 | 0,19, I (93:7) | 10 |
| 23 | Boc-Asn | 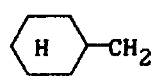 | 17 | 591 | 0,14, I (94:6) | 13 |

Fortsetzung Tabelle 2

| Beispiel-Nr. | W-A-B-D | $R^1$ | Ausbeute (%) | MS (FAB) m/z $(M+H)^+$ | $R_f$/Laufmittel- (Verhältnis) | Startmaterial aus Beispiel |
|---|---|---|---|---|---|---|
| 24 | $CH_3(CH_2)_{12}CO$-Phe | 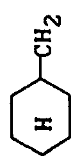 | 57 | 735 | 0,15, I (95:5) | 12 |
| 25 | Boc-Ser-Phe-Asn | 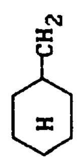 | 28 | 825 | 0,31, I (9:1) | 14 |

Beispiel 26

1-{(3S, 4S)-4-[(2S)-3-(tert.Butylsulfonyl)-2-(1-naphthylmethyl)propanoyl]valinylamino]-5-cyclohexyl-3-hydroxy-pentanoyl}-(2R oder 2S)-2-(pyrrolidinyl)phosphonsäurediethylester

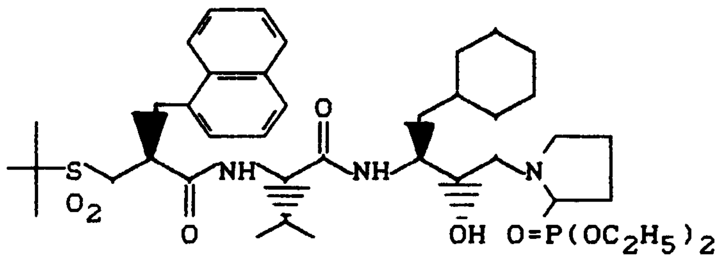

Eine auf 0° C gekühlte, gerührte Lösung von 126 mg (0,38 mmol) (2S)-3-tert.Butylsulfonyl-2-(1-napthylmethyl)-propionsäure [hergestellt nach H. Bühlmayer et al., J. Med. Chem. 31, 1839 (1988)] und 56 mg (0,42 mmol) HOBT in 4 ml wasserfreiem Dichlormethan wurde mit 82 mg (0,40 mmol) DCC versetzt und 5 min gerührt. Danach wurde eine Lösung von 188 mg (0,34 mmol) der Verbindung aus Beispiel 14a und 0,13 ml (1,19 mmol) N-Methylmorpholin in 3 ml Dichlormethan zugetropft und die Reaktion durfte 1 h bei Raumtemperatur rühren. Man trennte den entstandenen Harnstoff durch Filtration ab, engte das Filtrat im Vakuum ein und reinigte das Rohprodukt durch Chromatographie an 33 g Kieselgel (Dichlormethan : Methanol 95:5). Man erhielt 194 mg (72% der Theorie) der Titelverbindung als farblosen Schaum.
$R_f$ = 0,25, I (95:5) unpolares Diastereomer
MS (FAB): m/z = 792 $(M+H)^+$.

Wie für Beispiel 26 beschrieben, erhielt man durch Kupplung der entsprechenden Säuren mit den Aminhydrochloriden (Startmaterial) die folgenden Produkte (Tabelle 3):

<u>Tabelle 3</u>

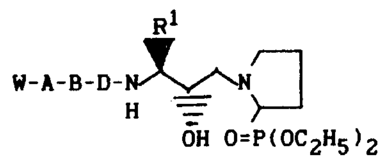

| Beispiel-Nr. | W-A-B-D- | $R^1$ | Ausbeute (%) | MS (FAB) m/z $(M+H)^+$ | $R_f$/Laufmittel- (Verhältnis) | Startmaterial aus Beispiel |
|---|---|---|---|---|---|---|
| 27 | 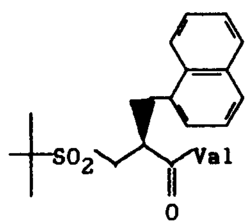 | 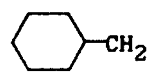 | 62 | 792 | 0,24, I (95:5) | 14b (polar) |
| 28 | 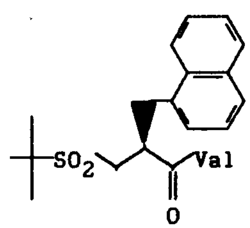 | $PhCH_2$ | 64 | 786 | 0,17, I (95:5) | 8 (unpolar) |

Forsetzung Tabelle 3

| Beispiel-Nr. | W-A-B-D- | $R^1$ | Ausbeute (%) | MS (FAB) m/z $(M+H)^+$ | $R_f$/Laufmittel- (Verhältnis) | Startmaterial aus Beispiel |
|---|---|---|---|---|---|---|
| 29 | 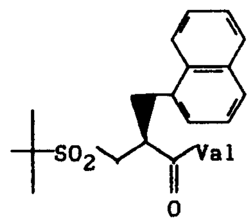 | $PhCH_2$ | 53 | 786 | 0,12, I (95:5) | 9 (polar) |
| 30 | 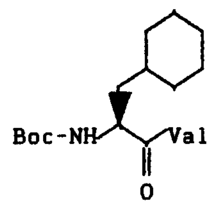 | $PhCH_2$ | 60 | 723 | 0,16, I (95:5) | 9 |
| 31 | Boc-Val | 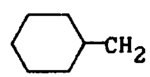 | 69 | 576 | 0,20, I (95:5) | 12 |
| 32 | Boc-Val | 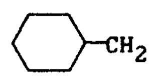 | 76 | 576 | 0,08, I (93:7) | 13 |
| 33 | Boc-Phe-Val | 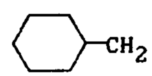 | 64 | 723 | 0,17, I (94:6) | 14a (unpolar) |

Forsetzung Tabelle 3

| Beispiel-Nr. | W-A-B-D- | $R^1$ | Ausbeute (%) | MS (FAB) m/z $(M+H)^+$ | $R_f$/Laufmittel- (Verhältnis) | Startmaterial aus Beispiel |
|---|---|---|---|---|---|---|
| 34 | Boc-Phe-Val | 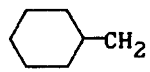 | 57 | 723 | 0,14, I (94:6) | 14b (polar) |
| 35 | Boc-Phe-Val | $PhCH_2$ | 63 | 717 | 0,23, I (95:5) | 8 (unpolar) |
| 36 | Boc-Phe-Val | $PhCH_2$ | 58 | 717 | 0,18, I (95:5) | 9 (polar) |
| 37 | 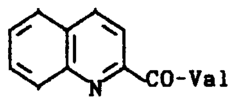 | $PhCH_2$ | 60 | 625 | 0,25, I (95:5) | 8 |
| 38 | 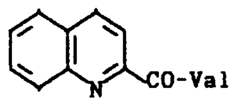 | $PhCH_2$ | 78 | 625 | 0,24, I (95:5) | 9 |
| 39 | 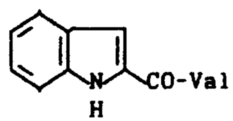 | $PhCH_2$ | 75 | 613 | 0,23, I (95:5) | 8 |
| 40 | 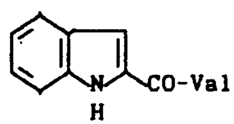 | $PhCH_2$ | 69 | 613 | 0,19, I (95:5) | 9 |

Beispiel 41

1-{(2R, 3S)-3-[(tert.Butoxycarbonyl)amino]-2-hydroxy-4-phenylbutyl}-(2R und 2S)-2-(pyrrolidinyl)-phosphonsäurediethylester

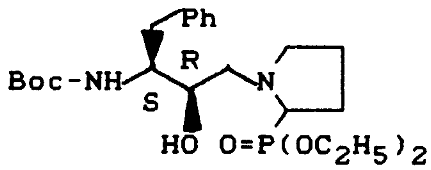

Wie für Beispiel 4 beschrieben erhielt man aus 877 mg (3,33 mmol) (1R,S)-[1'S-(tert.Butoxycarbonyl)-amino]-2-phenylethyl]oxiran [J.R. Luly et al., J. Org. Chem. 52, 487 (1987)] und 840 mg (4,0 mmol) (2-Pyrrolidinyl)phosphonsäurediethylester [US 4 186 268] und aufwendiger chromatographischer Trennung des Rohgemischs 198 mg (12% der Theorie) der Titelverbindung als Öl.
$R_f$ = 0,21, II (1:4)
MS (FAB): m/z = 471 $(M+H)^+$, 493 $(M+Na)^+$

Beispiel 42

1-[(2R, 3S)-3-(Amino)-2-hydroxy-4-phenylbutyl]-(2R und 2S)-2-(pyrrolidinyl)phosphonsäurediethylester Hydrochlorid

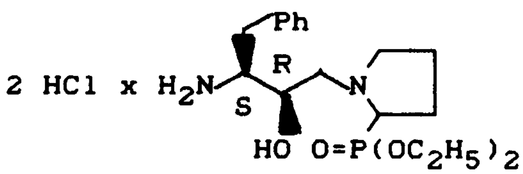

Wie für Beispiel 2 beschrieben erhielt man aus 1,286 g (2,73 mmol) der Verbindung aus Beispiel 42 925 mg (93% der Theorie) der Titelverbindung als amorphes Pulver.
$R_f$ = 0,50, I (8:2)
MS (FAB): m/z = 371 $(M+H)^+$, 393 $(M+Na)^+$

Beispiel 43

1-{(2R, 3S)-3-[(tert.Butoxycarbonyl)aspariginylamino]-2-hydroxy-4-phenyl-butyl}-(2R,S)-2-(pyrrolidinyl)-phosphonsäurediethylester

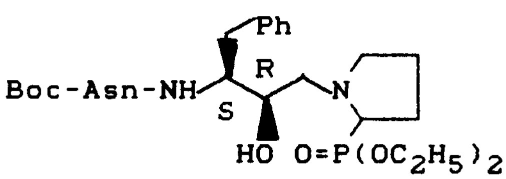

Wie für Beispiel 15 beschrieben erhielt man aus 892 mg (2,01 mmol) der Verbindung aus Beispiel 42 und 513 mg (2,21 mmol) N-(tert.Butoxycarbonyl)-L-Asparagin und Chromatographie des Rohprodukts an 140 g Kieselgel (Dichlormethan : Methanol, 9:1) 294 mg (33% der Theorie) der Titelverbindung als farblosen Schaum.
$R_f$ = 0,30, I (9:1)
MS (FAB): m/z = 585 $(M+H)^+$, 607 $(M+Na)^+$

Beispiel 44

1-[(2R, 3S)-3-(Asparaginylamino)-2-hydroxy-4-phenylbutyl]-(2R,S)-2-(pyrrolidinyl)phosphonsäurediethylester

Hydrochlorid

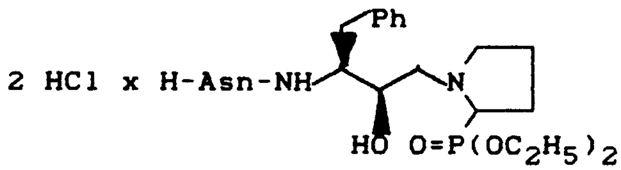

Wie für Beispiel 2 beschrieben erhielt man aus 210 mg (0,36 mmol) der Verbindung aus Beispiel 43 140 mg (69% der Theorie) der Titelverbindung als helles hygroskopisches Pulver.
Zersetzungspunkt 191° C
$R_f$ = 0,35, III (95:5)
MS (FAB): m/z = 485 $(M+H)^+$, 507 $(M+Na)^+$

Beispiel 45

1-{(2R, 3S)-3-[(2-Chinolylcarbonyl)asparaginylamino]-2-hydroxy-4-phenyl-butyl]-(2R,S)-2-(pyrrolidinyl)-phosphonsäurediethylester

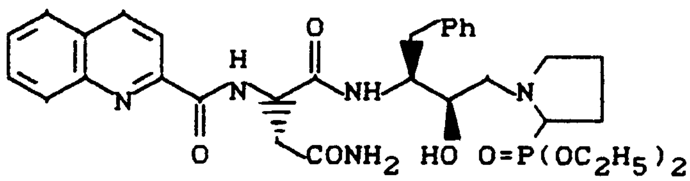

Wie für Beispiel 15 beschrieben erhielt man aus 120 mg (0,25 mmol) der Verbindung aus Beispiel 44 und 47 mg (0,26 mmol) Chinolin-2-carbonsäure und Chromatographie des Rohprodukts an 12 g Kieselgel (Dichlormethan : Methanol , 9:1) 69 mg (43% der Theorie) der Titelverbindung als farblosen Schaum.
$R_f$ = 0,14, I (9:1)
MS (FAB) m/z = 640 $(M+H)^+$

Experimenteller Teil II:

Ausgangsverbindungen

Beispiel I

(S)-2-Amino-1-phenylbut-3-en Hydrochlorid

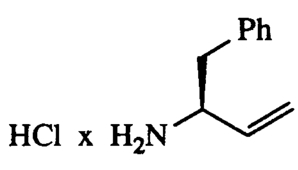

Eine Lösung von 5,00 g (20,21 mmol) (S)-2-(tert.Butoxycarbonylamino-1-phenylbut-3-en [J.R. Luly et al., J. Org. Chem. 52, 1487 (1987)] in 100 ml einer 4 N Lösung gasförmigen Chlorwasserstoffs in wasserfreiem Dioxan wurde 30 min bei Raumtemperatur gerührt. Danach gab man 15 ml Toluol zu und engte im Vakuum ein. Dieser Vorgang wurde noch zweimal wiederholt, dann wurde der Rückstand mit wenig Ether verrieben, abgesaugt und im Hochvakuum über KOH getrocknet. Man erhielt 3,69 g (99% der Theorie) der Titelverbindung als farblose Kristalle.
$R_f$ = 0,67, Laufmittelgemisch IV
MS (DCl, $NH_3$): m/z = 148 $(M+H)^+$

Beispiel II

(S)-2-Amino-1-cyclohexylbut-3-en Hydrochlorid

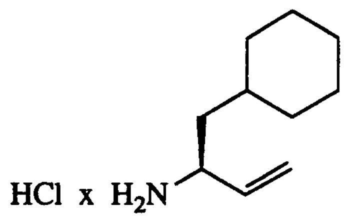

Wie für Beispiel I beschrieben erhielt man aus 5.07 g (20,00 mmol) (S)-2-(tert.Butoxycarbonylamino-1-cyclohexylbut-3-en [J.R. Luly et al., J. Org. Chem. 52, 1487 (1987)] 3,76 g (99% der Theorie) der Titelverbindung als farblose Kristalle.
Schmp.: 232-233 °C
$R_f$ = 0,42, III (9:1)
MS (EI, 70 eV) m/z = 153 $(M)^+$

Beispiel III

(2S)-2-[N-(tert.Butoxycarbonyl-L-valinyl)]amino-1-phenylbut-3-en

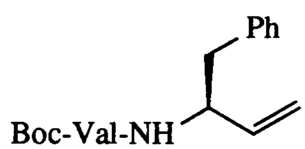

Eine auf 0 °C gekühlte, gerührte Lösung von 4,81 g (22,13 mmol) N-(tert.Butoxycarbonyl)-L-valin und 3,29 g (24,35 mmol) HOBT in 40 ml wasserfreiem Dichlormethan wurde mit 5,29 g (25,65 mmol) DCC versetzt und 5 min gerührt. Danach wurde eine Lösung von 3,70 g (20.12 mmol) der Verbindung aus Beispiel I und 8,85 ml (80,48 mmol) N-Methylmorpholin in 30 ml Dichlormethan zugetropft. Das Kühlbad wurde entfernt und die Reaktionsmischung durfte 2 h bei Raumtemperatur rühren. Das Ende der Reaktion wurde dünnschichtchromatographisch festgestellt. Man trennte den entstandenen Harnstoff durch Filtration ab, engte das Filtrat im Vakuum ein und reinigte das Rohprodukt durch Chromatographie an 450 g Kieselgel (Dichlormethan : Methanol 95:5). Man erhielt 6,07 g (87% der Theorie) der Titelverbindung als farblosen Schaum.
$R_f$ = 0,41, IV
MS (DCl, $NH_3$): m/z = 347 $(M+H)^+$.

Beispiel IV

(2S)-2-[N-(tert.Butoxycarbonyl)-L-valinyl)]amino-1-cyclohexylbut-3-en

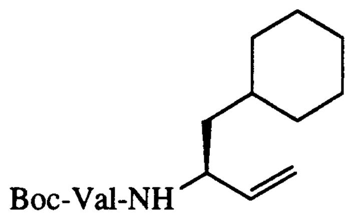

Wie für Beispiel III beschrieben erhielt man aus 3,60 g (19,00 mmol) der Verbindung aus Beispiel II und 4,63 g (21,3 mmol) Boc-Val-OH 4,33 g (65% der Theorie) der Titelverbindung als farblose Kristalle.
Schmp.: 127-128 °C (Zers.)
$R_f = 0,27$, II (9:1)
MS (DCI, $NH_3$) m/z = 353 $(M+H)^+$

Beispiel V

(2S)-1-Phenyl-2-(N-L-valinyl)aminobut-3-en Hydrochlorid

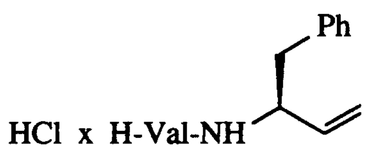

Wie für Beispiel I beschrieben erhielt man aus 6,08 g (17,53 mmol) der Verbindung aus Beispiel III 4,90 g (99% der Theorie) der Titelverbindung als farbloses Pulver.
$R_f = 0,36$, I (9:1)

Wie für Beispiel V beschrieben erhielt man die folgenden Hydrochloride:

Tabelle 4

HCl x H – A - B – D - N 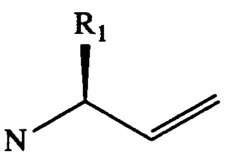

| Beispiel-Nr. | H-A-B-D- | $R^1$ | Ausbeute (%) | MS(FAB) m/z $(M+H)^+$ | $R_f$/Laufmittel Verhältnis | Startmaterial aus Beispiel |
|---|---|---|---|---|---|---|
| Va | H-Asn | $CH_2-C_6H_5$ | 95 | 262 | 0.06, I(9:1) | XXX |
| Vb | H-Aib | $CH_2-C_6H_5$ | 88 | 233 | 0.50, IV | XXVII |
| Vc | H-Ile | $CH_2-C_6H_5$ | 98 | 261 | 0,65, IV | XXX |

Beispiel VI

(2S)-1-Cyclohexyl-2-(N-L-valinyl)aminobut-3-en Hydrochlorid

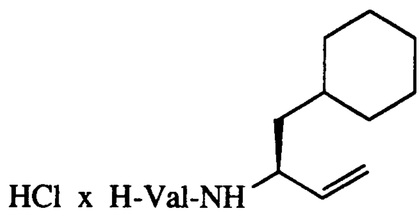

Wie für Beispiel I beschrieben erhielt man aus 4,32 g (12,30 mmol) der Verbindung aus Beispiel IV 3,37 g (95% der Theorie) der Titelverbindung als farbloses Pulver.
Schmp.: 169-170 °C
$R_f$ = 0,48, III (9:1)
MS (DCI, $NH_3$) m/z = 253 $(M+H)^+$

Beispiel VII

(2S)-2-[N-(2S)-3-(tert.Butylsulfonyl)-2-(1-naphthylmethyl)propanoyl]-L-valinyl]amino-1-phenylbut-3-en

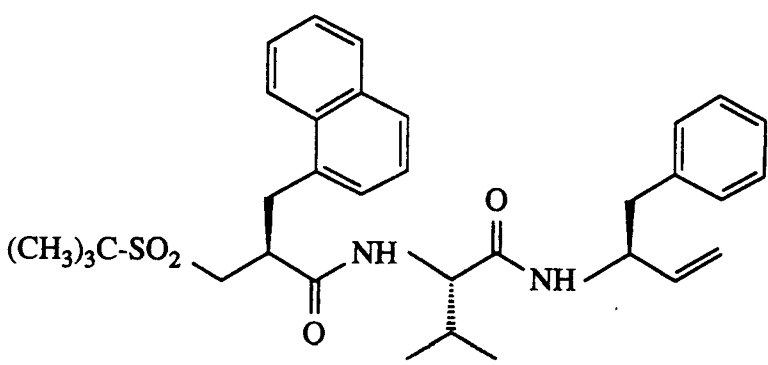

Eine auf 0 °C gekühlte, gerührte Lösung von 1,50 g (4,47 mmol) (2S)-3-tert.Butylsulfonyl-2-(1-naphthylmethyl)-propionsäure [hergestellt nach H. Bühlmayer et al., J. Med. Chem. 31, 1839 (1988)] und 0,66 g (4,92 mmol) HOBT in 15 ml wasserfreiem Dichlormethan wurde mit 0,97 g (4,69 mmol) DCC versetzt und 5 min gerührt. Danach wurde eine Lösung von 1,15 g (4,07 mmol) der Verbindung aus Beispiel V und 1,80 ml (16,27 mmol) N-Methylmorpholin in 10 ml Dichlormethan zugetropft und die Reaktion durfte 1 h bei Raumtemperatur rühren. Man trennte den entstandenen Harnstoff durch Filtration ab, engte das Filtrat im Vakuum ein und reinigte das Rohprodukt durch Chromatographie an 270 g Kieselgel (Dichlormethan : Methanol 95:5). Man erhielt 2.01 g (88% der Theorie) der Titelverbindung als farblosen Schaum.
$R_f$ = 0,47, I (95:5)
MS (FAB) m/z = 563 $(M+H)^+$

Wie für Beispiel VII beschrieben, erhielt man durch Kupplung der entsprechenden Säuren mit den Aminhydrochloriden (Startmaterialien) die folgenden Produkte (Tabelle 5):

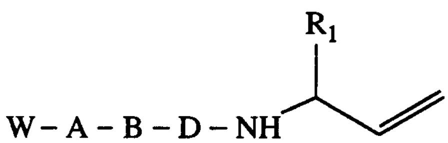

Tabelle 5

| Beispiel-Nr. | W-A-B-D- | $R^1$ | Ausbeute (%) | MS(FAB) m/z $(M+H)^+$ | $R_f$/Laufmittel Verhältnis | Startmaterial aus Beispiel |
|---|---|---|---|---|---|---|
| VIII | 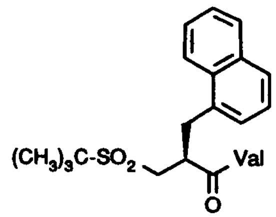 | $C_6H_{11}$-$CH_2$ | 76 | 569 | 0,57, I (1:1) | VI |
| IX | 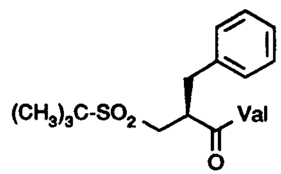 | $C_6H_5$-$CH_2$ | 92 | 513 | 0,48, I (95:5) | V |
| X | 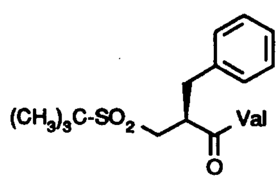 | $C_6H_{11}$-$CH_2$ | 75 | 519 | 0,56, II (1:1) | VI |

| Beispiel-Nr. | W-A-B-D- | $R^1$ | Ausbeute (%) | MS(FAB) m/z $(M+H)^+$ | $R_f$/Laufmittel Verhältnis | Startmaterial aus Beispiel |
|---|---|---|---|---|---|---|
| XI | $CH_3$-$SO_2$-Phe-Val | $CH_2$-$C_6H_5$ | 62 | 472 | 0,12, I (95:5) | V |
| XII | $CH_3$-$SO_2$-Phe-Val | $CH_2$-$C_6H_{11}$ | 58 | 478 | 0,50, II (1:1) | VI |
| XIII | 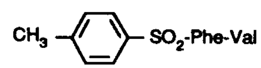 | $CH_2$-$C_6H_5$ | 29 | 548 | 0,53, I (95.5) | V |
| XIV | 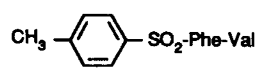 | $CH_2$-$C_6H_{11}$ | 21 | 554 | 0,70, II (1:1) | VI |
| XV | $(CH_3)_3C$-$CH_2$-CO-Phe-Val | $CH_2$-$C_6H_5$ | 80 | 492 | 0,31, I (95:5) | V |
| XVI | $(CH_3)_3C$-$CH_2$-CO-Phe-Val | $CH_2$-$C_6H_{11}$ | 57 | 498 | 0,25, I (95:5) | VI |

| Beispiel-Nr. | W-A-B-D- | $R^1$ | Ausbeute (%) | MS(FAB) m/z $(M+H)^+$ | $R_f$/Laufmittel Verhältnis | Startmaterial aus Beispiel |
|---|---|---|---|---|---|---|
| XVII | Boc-Phe-Val | $CH_2$-$C_6H_5$ | 74 | 494 | 0,44, I (95:5) | V |
| XVIII | Boc-Phe-Val | $CH_2$-$C_6H_{11}$ | 62 | 500 | 0,38, I (95:5) | VI |
| XIX | Z-Phe-Val | $CH_2$-$C_6H_5$ | 65 | 528 | 0,56, I (95:5) | V |
| XX | Z-Phe-Val | $CH_2$-$C_6H_{11}$ | 75 | 534 | 0,25, I (95:5) | VI |
| XXI | 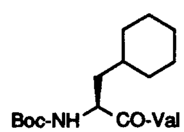 | $CH_2$-$C_6H_5$ | 84 | 500 | 0,43, I (95:5) | V |

| Beispiel-Nr. | W-A-B-D- | $R^1$ | Ausbeute (%) | MS(FAB) m/z $(M+H)^+$ | $R_f$/Laufmittel Verhältnis | Startmaterial aus Beispiel |
|---|---|---|---|---|---|---|
| XXII | 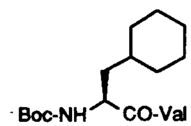 | $CH_2$-$C_6H_{11}$ | 70 | 506 | 0,38, I (95:5) | VI |
| XXIII | $CH_3(CH_2)_{12}$-CO-Phe-Val | $CH_2$-$C_6H_5$ | 82 | 604 | 0,34, I (95:5) | V |
| XXIV | 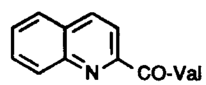 | $CH_2$-$C_6H_5$ | 54 | 402 | 0,61, I (95:5) | V |
| XXV | 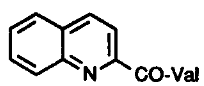 | $CH_2$-$C_6H_{11}$ | 54 | 408 | 0,21, II (4:1) | VI |
| XXVI | 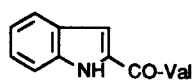 | $CH_2C_6H_5$ | 96 | 390 | 0,51, I (95:5) | V |

| Beispiel-Nr. | W-A-B-D- | $R^1$ | Ausbeute (%) | MS(FAB) m/z $(M+H)^+$ | $R_f$/Laufmittel Verhältnis | Startmaterial aus Beispiel |
|---|---|---|---|---|---|---|
| XXVII | Boc-Aib | $CH_2$-$C_6H_5$ | 52 | 333 | 0,51, I (97:3) | V |
| XXVIII | Boc-Phe-Gly-Gly | $CH_2$-$C_6H_5$ | 77 | 509 | 0,45, IV | V |
| XXIX | Boc-Ser-Phe | $CH_2$-$C_6H_5$ | 34 | 482 | 0,45, I (9:1) | V |
| XXX | Boc-Asn | $CH_2$-$C_6H_5$ | 38 | 362 | 0,13, I (95:5) | V |
| XXXI | Boc-Ile | $CH_2$-$C_6H_5$ | 64 | 361 | 0,58, I (97:3) | V |
| XXXII | 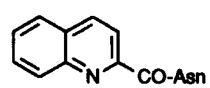 | $CH_2$-$C_6H_5$ | 39 | 417 | 0,26, I (95:5) | Va |
| XXXIII | 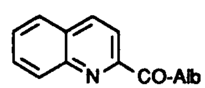 | $CH_2$-$C_6H_5$ | 70 | 388 | 0,48, II (7:3) | Vb |

| Beispiel-Nr. | W-A-B-D- | $R^1$ | Ausbeute (%) | MS(FAB) m/z $(M+H)^+$ | $R_f$/Laufmittel Verhältnis | Startmaterial aus Beispiel |
|---|---|---|---|---|---|---|
| XXXIV | 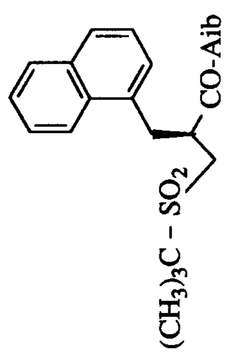 | $CH_2$-$C_6H_5$ | 41 | 549 | 0,35, II (3:2) | Vb |
| XXXV | 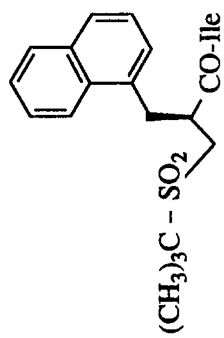 | $CH_2$-$C_6H_5$ | 72 | 578 | 0,18, II (7:3) | Vc |
| XXXVI | 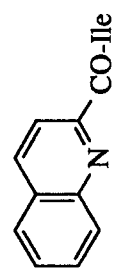 | $CH_2$-$C_6H_5$ | 94 | 416 | 0,28, II (4:1) | Vc |

Herstellungsbeispiele

Beispiel 46

2-{1-[N-[(tert.Butylacetyl)-L-phenylalanyl]-L-valinyl]amino-2-phenyl-(1S)-ethyl}oxiran

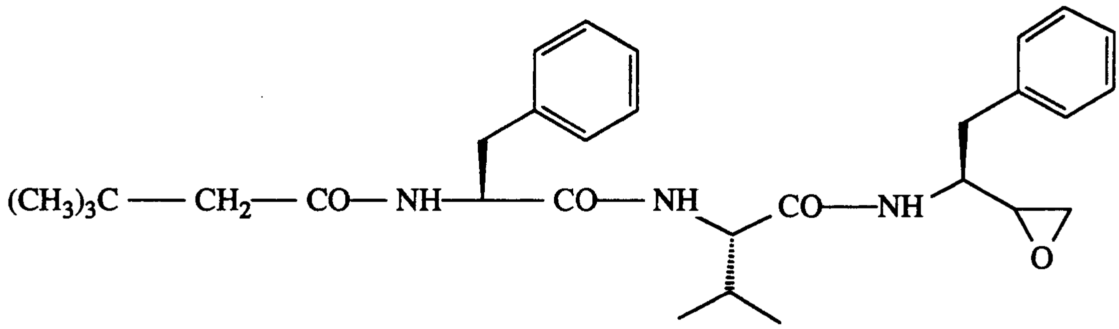

Eine gerührte, auf 0 °C gekühlte Suspension von 250 mg (0,60 mmol) der Verbindung aus Beispiel XV in 3 ml Dichlormethan wurde portionsweise mit 259 mg (1,20 mmol - 2 equiv.) m-Chlorperbenzoesäure (80%ig) (MCPBA) versetzt und 2 h bei dieser Temperatur gerührt. Danach wurden weitere 130 mg (0,60 mmol - 1 equiv.) MCPBA zugegeben und noch 1 h bei Raumtemperatur nachgerührt. Danach wurden 10 ml Ethylacetat zugegeben und die Reaktionsmischung wurde in 20 ml einer 10%igen $Na_2SO_3$-Lösung eingerührt. Die organische Phase wurde abgetrennt, 3 mal mit 10 ml $NaHCO_3$-Lösung gewaschen und über $MgSO_4$ getrocknet. Nach Abdampfen des Lösemittels im Vakuum und Verreiben des Rückstandes mit wenig Ether/Pentan erhielt man die 253 mg (83% der Theorie) der Titelverbindung als farbloses Pulver.

Schmp.: 168 °C (Zers.)

$R_f$ = 0,26, I (97:3)

MS (FAB) m/z = 508 $(M+H)^+$

Beispiel 47

(2S)-{1-[N-[(2S)-Benzyl-3-(tert.Butylsulfonyl)propanoyl]-L-valinyl]amino-2-phenyl-(1S)-ethyl}oxiran

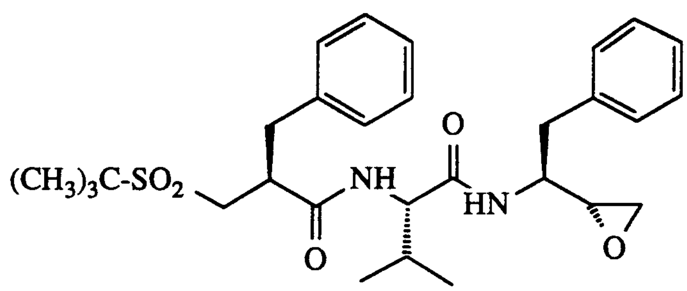

Eine Suspension von 345 mg (0,67 mmol) der Verbindung aus Beispiel IX, 8 mg (5 mol-%) Benzyltriethylammoniumchlorid und 668 mg (1,35 mmol) Magnesiummonoperoxyphthalat Hexahydrat (MMPP) in 3 ml Chloroform wurde durch Zugabe von 1N NaOH-Lösung auf pH 5 gestellt und 16 h zum Rückfluß erhitzt, wobei durch Zugabe kleiner Mengen von 1N NaOH etwa pH 5 gehalten wurde. Nach dem Abkühlen wurde die Reaktionsmischung abgesaugt und das Filtrat mit 10 ml Wasser, 10 ml 10%ige $Na_2SO_3$-Lösung und 10 ml verdünnte $NaHCO_3$-Lösung gewaschen und über Magnesiumsulfat getrocknet. Nach Abdampfen des Lösemittels im Vakuum und Chromatographie des Rückstands an 15 g Kieselgel (Toluol : Ethylacetat 1:1) erhielt man 131 mg (37% der Theorie) der Titelverbindung als farblosen Hartschaum.

$R_f$ = 0,21, II (1:1)

MS (FAB) m/z = 529 $(M+H)^+$

HPLC: Gemisch der diastereomeren Epoxide (1S): (1R) = 16:1

Wie für Beispiel 46 beschrieben, erhielt man die folgenden Epoxide (Tabelle 6):

Tabelle 6:

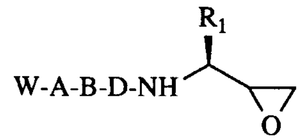

| Beispiel-Nr. | W-A-B-D- | $R^1$ | Ausbeute (%) | MS(FAB) m/z $(M+H)^+$ | $R_f$/Laufmittel Verhältnis | Startmaterial aus Beispiel |
|---|---|---|---|---|---|---|
| 48 | Z-Phe-Val | $CH_2-C_6H_5$ | 81 | 544 | 0,39 , I (95:5) | XIX |
| 49 | Boc-Cha-Val | $CH_2-C_6H_5$ | 47 | 516 | 0,53, I (95:5) | XXI |
| 50 | 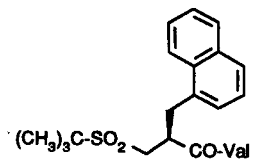 | $CH_2-C_6H_5$ | 4 | 579 | 0,16, II (4:6) | VII |
| 51 | 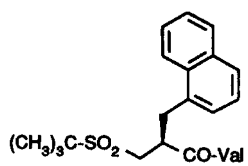 | $CH_2-C_6H_5$ | 38 | 579 | 0,12, II (4:6) | VII |

Fortsetzung Tabelle 6:

| Beispiel-Nr. | W-A-B-D- | $R^1$ | Ausbeute (%) | MS(FAB) m/z $(M+H)^+$ | $R_f$/Laufmittel Verhältnis | Startmaterial aus Beispiel |
|---|---|---|---|---|---|---|
| 52 | 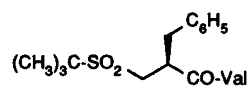 | $CH_2$-$C_6H_5$ | 43 | 529 | 0,21, II (1:1) | IX |
| 53 | $CH_3$-$SO_2$-Phe-Val | $CH_2$-$C_6H_5$ | 10 | 488 | 0,22, II (1:1) | |
| | | | | | | XI |
| 54 | $CH_3$-$SO_2$-Phe-Val | $CH_2$-$C_6H_5$ | 39 | 488 | 0,15, II (1:1) | |
| 55 | Boc-Phe-Val | $CH_2$-$C_6H_5$ | 46 | 510 | 0,05, I (97:3) | XVII |

Fortsetzung Tabelle 6:

| Beispiel-Nr. | W-A-B-D- | $R^1$ | Ausbeute (%) | MS(FAB) m/z $(M+H)^+$ | $R_f$/Laufmittel Verhältnis | Startmaterial aus Beispiel |
|---|---|---|---|---|---|---|
| 56 | 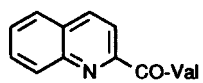 | $CH_2-C_6H_5$ | 66 | 418 | 0,25, II (7:3) | XXIV |
| 57 | 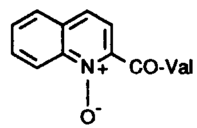 | $CH_2-C_6H_5$ | 22 | 418 | 0,12, II (7:3) | |
| 58 | $CH_3(CH_2)_{12}$-CO-Phe-Val | $CH_2-C_6H_5$ | 55 | 620 | 0,33, I (97:3) | XXIII |
| 59 | 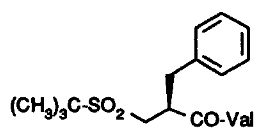 | $CH_2-C_6H_{11}$ | 54 | 535 | 0,12, I (95:5) | X |
| 60 | 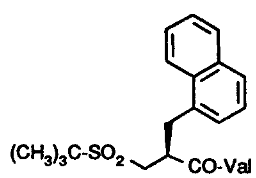 | $CH_2-C_6H_{11}$ | 53 | 585 | 0,28, I (97:3) | VIII |

Fortsetzung Tabelle 6:

| Beispiel-Nr. | W-A-B-D- | $R^1$ | Ausbeute (%) | MS(FAB) m/z $(M+H)^+$ | $R_f$/Laufmittel Verhältnis | Startmaterial aus Beispiel |
|---|---|---|---|---|---|---|
| 61 | Boc-Phe-Val | $CH_2$-$C_6H_{11}$ | 51 | 516 | 0,11, I (97:3) | XVIII |
| 62 | $CH_3$-$SO_2$-Phe-Val | $CH_2$-$C_6H_{11}$ | 55 | 494 | 0,13, I (97:3) | XII |
| 63 | $(CH_3)_3C$-$CH_2$-CO-Phe-Val | $CH_2$-$C_6H_{11}$ | 48 | 514 | 0,12, I (97:3) | XVI |
| 64 | Boc-Cha-Val | $CH_2$-$C_6H_{11}$ | 55 | 522 | 0,19, I (97:3) | XXII |
| 65 | Z-Phe-Val | $CH_2$-$C_6H_{11}$ | 61 | 550 | 0,34, II (6:4) | XX |
| 66 | Z-Phe-Val | $CH_2$-$C_6H_{11}$ | 8 | 550 | 0,14, II (6:4) | XX |

Fortsetzung Tabelle 6:

| Beispiel-Nr. | W-A-B-D- | $R^1$ | Ausbeute (%) | MS(FAB) m/z $(M+H)^+$ | $R_f$/Laufmittel Verhältnis | Startmaterial aus Beispiel |
|---|---|---|---|---|---|---|
| 67 | Boc-Ser-Phe | $CH_2$-$C_6H_5$ | 38 | 500 | 0,12, I (9:1) | XXIX |
| 68 | Boc-Phe-Gly-Gly | $CH_2$-$C_6H_5$ | 49 | 525 | 0,41, I (9:1) | XXVIII |
| 69 | 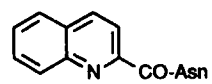 | $CH_2$-$C_6H_5$ | 17 | 433 | 0,11, I (95:5) | XXXII |
| 70 | 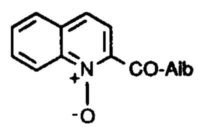 | $CH_2$-$C_6H_5$ | 55 | 420 | 0,13, II (3:2) | XXXIII |
| 71 | 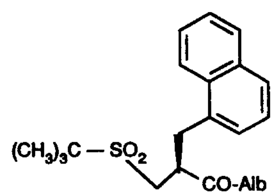 | $CH_2$-$C_6H_5$ | 42 | 565 | 0,13, I (93:3) | XXXIV |
| 72 | 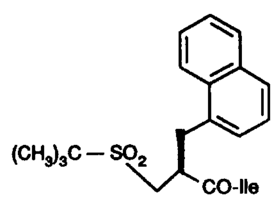 | $CH_2$-$C_6H_5$ | 53 | 594 | 0,18, II (2:3) | XXXV |

Beispiel 73

2-{1-[N-[(2S)-3-(tert.Butylsulfonyl)-2-(1-naphthylmethyl)propanoyl]-L-valinyl]-amino-2-phenyl-(1S)-ethyl}thiiran

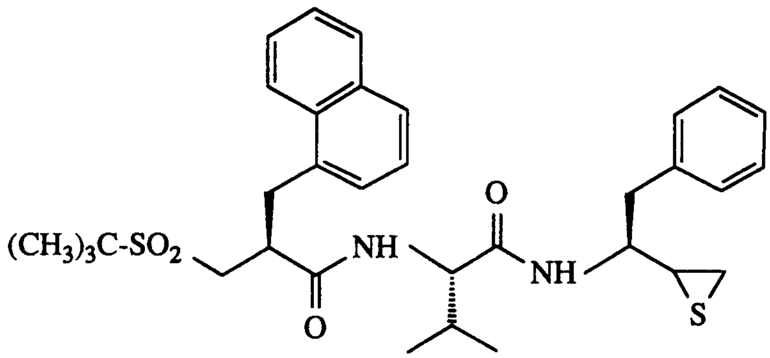

Eine gerührte Lösung von 159 mg (0,27 mmol) der Verbindung aus Beispiel 51 (polares Isomer) 47 μl (0.54 mmol - 2 equiv.) Thiodimethylformamid und 2 μl (0,027 mmol - 0,1 equiv.) Trifluoressigsäure in 1 ml wasserfreiem Dichlorethan (Herstellung via Maßlösung) wurde 1 h auf 60 °C Badtemperatur erwärmt. Danach wurde das Reaktionsgemisch mit 10 ml Ethylacetat verdünnt und in einem Gemisch aus 10 ml Ethylacetat, 10 ml $NaHCO_3$-Lösung und 10 ml NaCl-Lösung eingerührt. Die organische Phase wurde abgetrennt, mit einem Gemisch aus 10 ml $NaHCO_3$ : NaCl-Lösung (1:1) gewaschen und über $MgSO_4$ getrocknet. Nach Abdampfen des Lösemittels und Chromatographie des Rohproduktes an 11 g Kieselgel (Dichlormethan : Methanol 95:5) und dann an 6 g Kieselgel (Toluol : Ethylacetat 4:6) erhielt man die Titelverbindung als farblosen Schaum.

$R_f$ = 0,25, I (95:5)

MS (FAB) m/z = 595 $(M+H)^+$

Wie für Beispiel 73 beschrieben erhielt man durch Umsetzung der entsprechenden Epoxide die folgenden Thiirane (Tabelle 7):

Tabelle 7:

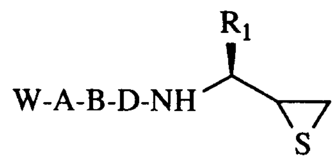

| Beispiel-Nr. | W-A-B-D- | $R^1$ | Ausbeute (%) | MS(FAB) m/z $(M+H)^+$ | $R_f$/Laufmittel Verhältnis | Startmaterial aus Beispiel |
|---|---|---|---|---|---|---|
| 74 | 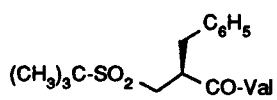 | $CH_2$-$C_6H_{11}$ | 21 | 551 | 0,14, I (97:3) | 59 |
| 75 | 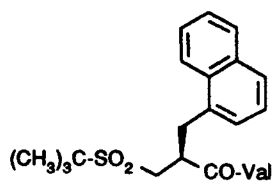 | $CH_2$-$C_6H_{11}$ | 31 | 601 | 0,27, II (6:4) | 60 |
| 76 | $CH_3$-$SO_2$-Phe-Val | $CH_2$-$C_6H_{11}$ | 29 | 510 | 0,17, I (95:5) | 62 |

Fortsetzung Tabelle 7:

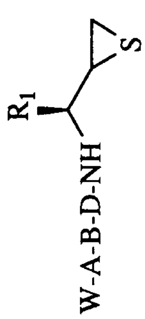

| Beispiel-Nr. | W-A-B-D- | $R^1$ | Ausbeute (%) | MS(FAB) m/z $(M+H)^+$ | $R_f$/Laufmittel Verhältnis | Startmaterial aus Beispiel |
|---|---|---|---|---|---|---|
| 77 | 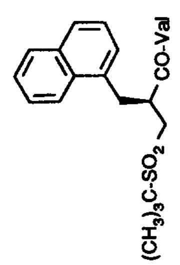 | $CH_2-C_6H_5$ | 24 | 595 | 0,25, I (95:5) | 50 |
| 78 | Boc-Cha-Val | $CH_2-C_6H_5$ | 36 | 538 | 0,35, I (95:5) | 64 |

## Patentansprüche

**1.** Phosphonat-haltige Pseudopeptide der allgemeinen Formel (I),

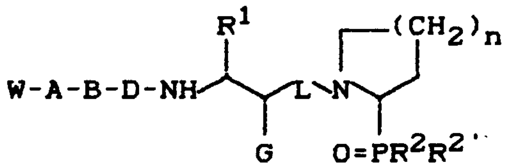 (I)

in welcher

W

- für Wasserstoff oder für eine typische Aminoschutzgruppe steht, oder
- für geradkettiges oder verzweigtes Alkyl oder Alkenyl mit jeweils bis zu 6 Kohlenstoffatomen steht, die gegebenenfalls durch Aryl mit 6 bis 10 Kohlenstoffatomen substituiert sind, oder
- für eine Gruppe der Formel $R^3$-CO-, $R^5R^4$N-CO- oder $R^6$-$SO_2$- steht,

worin

$R^3$

- Wasserstoff, Trifluormethyl oder geradkettiges oder verzweigtes Alkoxy mit bis zu 8 Kohlenstoffatomen oder Alkyl mit bis zu 18 Kohlenstoffatomen bedeutet, das gegebenenfalls bis zu 2-fach durch Aryl mit 6 bis 10 Kohlenstoffatomen oder Pyridyl substituiert ist, oder
- Aryl mit 6 bis 10 Kohlenstoffatomen bedeutet, das gegebenenfalls durch Halogen, Trifluormethyl, Trifluormethoxy oder durch geradkettiges oder verzweigtes Alkyl mit bis zu 8 Kohlenstoffatomen substituiert ist,
- Cycloalkyl mit 3 bis 7 Kohlenstoffatomen bedeutet, oder
- Chinolyl, Chinolyl-N-oxid, Indolyl, Pyridyl, Pyridyl-N-oxid, Morpholino oder Piperazinyl bedeutet, oder
- einen Rest der Formel

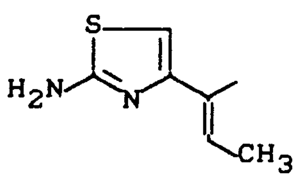 , 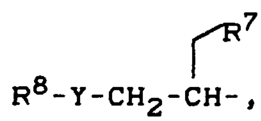

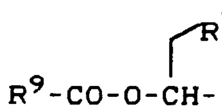 , 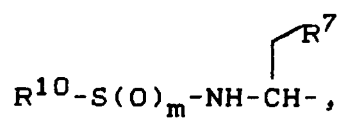

T-NH-$(CH_2)_p$- , 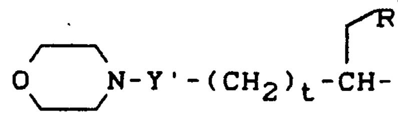

oder 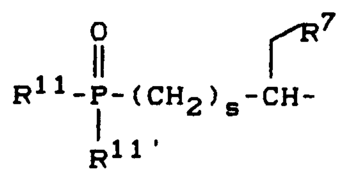

bedeutet,

worin

$R^7$ - Phenyl oder Naphthyl bedeutet,

$R^8$, $R^9$ und $R^{10}$ gleich oder verschieden sind sind und geradkettiges oder verzweigtes Alkyl mit bis zu 14 Kohlenstoffatomen bedeuten, das gegebenenfalls durch Phenyl oder Naphthyl substituiert ist, oder Aryl mit 6 bis 10 Kohlenstoffatomen bedeuten, welches seinerseits durch Alkyl mit bis zu 4 Kohlenstoffatomen substituiert ist,

m - eine Zahl 0, 1 oder 2 bedeutet,

T - Cyclohexyl bedeutet,

p - eine Zahl 1, 2 oder 3 bedeutet,

Y und Y' gleich oder verschieden sind und die CO- oder $SO_2$-Gruppe bedeuten,

t - eine Zahl 0 oder 1 bedeutet,

$R^{11}$ und $R^{11'}$ gleich oder verschieden sind und Hydroxy oder Alkoxy mit bis zu 8 Kohlenstoffatomen bedeuten,

s - eine Zahl 1 oder 2 bedeutet,

$R^4$ und $R^5$ gleich oder verschieden sind und
- Wasserstoff oder
- Aryl mit 6 bis 10 Kohlenstoffatomen bedeuten, das gegebenenfalls durch geradkettiges oder verzweigtes Alkyl mit bis zu 6 Kohlenstoffatomen oder Halogen substituiert ist, oder
- Cycloalkyl mit 3 bis 7 Kohlenstoffatomen bedeuten, oder
- geradkettiges oder verzweigtes Alkyl mit bis zu 18 Kohlenstoffatomen bedeuten,

$R^6$ - geradkettiges oder verzweigtes Alkyl mit bis zu 8 Kohlenstoffatomen bedeutet,

A, B und D gleich oder verschieden sind und
- für eine direkte Bindung oder
- für einen Rest der Formel

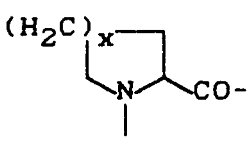

oder

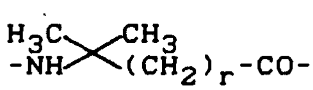

stehen,

worin

x - die Zahl 1 oder 2 bedeutet

und

r - die Zahl 0 oder 1 bedeutet,

oder
- für eine Gruppe der Formel

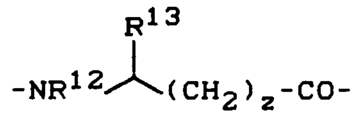

stehen

worin

z - die Zahl 0 oder 1 bedeutet,

$R^{12}$ Wasserstoff oder geradkettiges oder verzweigtes Alkyl mit bis zu 4 Kohlenstoffatomen bedeutet,

$R^{13}$ Cycloalkyl mit 3 bis 8 Kohlenstoffatomen oder Aryl mit 6 bis 10 Kohlenstoffatomen oder Wasserstoff bedeutet, oder

- geradkettiges oder verzweigtes Alkyl mit bis zu 8 Kohlenstoffatomen bedeutet, das gegebenenfalls durch Methylthio, Hydroxy, Mercapto, Guanidyl oder durch eine Gruppe der Formel $-NR^{14}R^{15}$ oder $R^{16}$-OC-substituiert ist,

  worin

  $R^{14}$ und $R^{15}$ gleich oder verschieden sind und Wasserstoff, geradkettiges oder verzweigtes Alkyl mit bis zu 8 Kohlenstoffatomen oder Phenyl bedeuten

  und

  $R^{16}$ Hydroxy, Benzyloxy, Alkoxy mit bis zu 6 Kohlenstoffatomen oder die oben aufgeführte Gruppe $-NR^{14}R^{15}$ bedeutet,

oder das gegebenenfalls durch Cycloalkyl mit 3 bis 8 Kohlenstoffatomen oder durch Aryl mit 6 bis 10 Kohlenstoffatomen substituiert ist, das seinerseits durch Hydroxy, Halogen, Nitro, Alkoxy mit bis zu 8 Kohlenstoffatomen oder durch die Gruppe $-NR^{14}R^{15}$ substituiert ist,

worin

$R^{14}$ und $R^{15}$ die oben angegebene Bedeutung haben,

oder das gegebenenfalls durch einen 5- oder 6-gliedrigen stickstoffhaltigen Heterocyclus oder Indolyl substituiert ist,

worin die entsprechenden -NH-Funktionen gegebenenfalls durch Alkyl mit bis zu 6 Kohlenstoffatomen oder durch eine Aminoschutzgruppe geschützt sind,

$R^1$ - für geradkettiges oder verzweigtes Alkyl oder Alkenyl mit bis zu 10 Kohlenstoffatomen steht, die gegebenenfalls durch Cycloalkyl mit 3 bis 8 Kohlenstoffatomen oder Aryl mit 6 bis 10 Kohlenstoffatomen substituiert sind, das seinerseits durch Halogen, Nitro, Hydroxy, Amino oder geradkettiges oder verzweigtes Alkoxy mit bis zu 4 Kohlenstoffatomen substituiert sein kann,

n - für die Zahl 1 oder 2 steht,

$R^2$ und $R^{2'}$ gleich oder verschieden sind

- für Hydroxy, oder
- für geradkettiges oder verzweigtes Alkoxy mit bis zu 4 Kohlenstoffatomen stehen,

G - für eine Gruppe der Formel -SH oder -OH steht

und

L - für die $-CH_2$ oder -CO-Gruppe steht

und deren physiologisch unbedenklichen Salze.

**2.** Verbindungen der allgemeinen Formel (I) gemäß Anspruch 1

in welcher

W

- für Wasserstoff, tert.Butyloxycarbonyl (BOC), 9-Fluorenylmethyloxycarbonyl (Fmoc) oder Benzyloxycarbonyl (Z) steht, oder
- für geradkettiges oder verzweigtes Alkyl oder Alkenyl mit jeweils bis zu 4 Kohlenstoffatomen steht, die gegebenenfalls durch Phenyl substituiert sind, oder
- für eine Gruppe der Formel $R^3$-CO-, $R^5R^4N$-CO- oder $R^6-SO_2$ steht,

worin

$R^3$

- Wasserstoff, Trifluormethyl oder geradkettiges oder verzweigtes Alkoxy mit bis zu 4 Kohlenstoffatomen oder Alkyl mit bis zu 16 Kohlenstoffatomen bedeutet, das gegebenenfalls bis zu 2-fach durch Phenyl, Naphthyl oder Pyridyl substituiert ist, oder
- Phenyl oder Naphthyl bedeutet, die gegebenenfalls durch Fluor, Chlor, Trifluormethyl, Trifluormethoxy oder durch geradkettiges oder verzweigtes Alkyl mit bis zu 6 Kohlenstoffatomen substituiert sind,
- Cyclopropyl, Cyclopentyl, Cyclohexyl, Chinolyl, Chinolyl-N-oxid, Indolyl, Pyridyl, Pyridyl-N-oxid, Morpholino oder Piperazinyl bedeutet, oder
- einen Rest der Formel

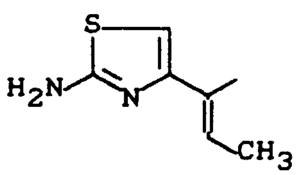 , 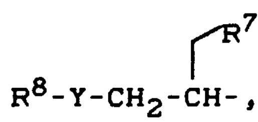,

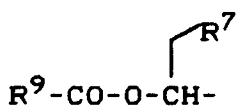 oder 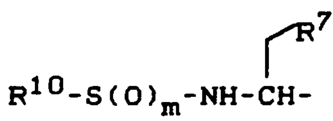

bedeutet,

worin

Y - die CO- oder $SO_2$-Gruppe bedeutet,

$R^7$ - Phenyl oder Naphthyl bedeutet,

$R^8$, $R^9$ und $R^{10}$ gleich oder verschieden sind

und geradkettiges oder verzweigtes Alkyl mit bis zu 8 Kohlenstoffatomen, Tolyl, Phenyl oder Naphthyl bedeuten,

m - eine Zahl 1 oder 2 bedeutet,

$R^4$ und $R^5$ gleich oder verschieden sind und

- Wasserstoff oder

- Phenyl oder Naphthyl bedeuten, die gegebenenfalls durch geradkettiges oder verzweigtes Alkyl mit bis zu 4 Kohlenstoffatomen, Fluor oder Chlor substituiert sind,
- Cyclopropyl, Cyclopentyl oder Cyclohexyl bedeuten, oder
- geradkettiges oder verzweigtes Alkyl mit bis zu 16 Kohlenstoffatomen bedeuten,

$R^6$ - geradkettiges oder verzweigtes Alkyl mit bis zu 6 Kohlenstoffatomen bedeutet,

A, B und D gleich oder verschieden sind und

- für eine direkte Bindung oder
- für Prolin stehen, oder
- für einen Rest der Formel

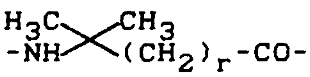

stehen,

worin

r die Zahl 0 oder 1 bedeutet

- für eine Gruppe der Formel

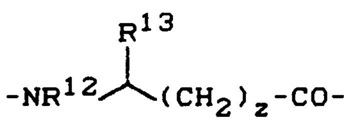

stehen

worin

z - die Zahl 0 oder 1 bedeutet,

$R^{12}$ - Wasserstoff, Methly oder Ethyl bedeutet,

$R^{13}$

- Cyclopentyl, Cyclohexyl, Phenyl oder Wasserstoff bedeutet,
- oder geradkettiges oder verzweigtes Alkyl mit bis zu 6 Kohlenstoffatomen bedeutet, das gegebenenfalls durch Methylthio, Hydroxy, Mercapto, Guanidyl, Amino, Carboxy oder $H_2N$-CO-substituiert sein kann,

  oder durch Cyclohexyl, Naphthyl oder Phenyl substituiert ist, das seinerseits durch Fluor, Chlor, Hydroxy, Nitro oder Alkoxy mit bis zu 4 Kohlenstoffatomen substituiert sein kann,

oder durch Indolyl, Imidazolyl, Pyridyl, Triazolyl oder Pyrazolyl substituiert ist, wobei die entsprechenden -NH-Funktionen gegebenenfalls durch Alkyl mit bis zu 4 Kohlenstoffatomen oder durch eine Aminoschutzgruppe geschützt sind,

$R^1$ - für geradkettiges oder verzweigtes Alkyl oder Alkenyl mit bis zu 8 Kohlenstoffatomen steht, die gegebenenfalls durch Cyclopropyl, Cyclobutyl, Cyclopentyl, Cyclohexyl, Cycloheptyl oder Phenyl substituiert sind, das seinerseits durch Fluor, Chlor, Brom, Nitro, Hydroxy oder Amino substituiert sein kann,

n - für die Zahl 1 oder 2 steht,

$R^2$ und $R^{2'}$ gleich oder verschieden sind und

- für Hydroxy, oder

- für geradkettiges oder verzweigtes Alkoxy mit bis zu 4 Kohlenstoffatomen stehen,

G - für eine Gruppe der Formel -SH oder -OH steht

und

L - für die $-CH_2$ oder -CO-Gruppe steht

und deren physiologisch unbedenklichen Salze.

**3.** Verbindungen der allgemeinen Formel (I) gemäß Anspruch 1,

in welcher

W

- für Wasserstoff, tert.Butyloxycarbonyl (BOC) oder Benzyloycarbonyl (Z) steht, oder
- für Allyl oder Benzyl steht,
- für eine Gruppe der Formel $R^3$-CO-, $R^5R^4$N-CO- oder $R^6-SO_2$ steht, worin

$R^3$

- Wasserstoff, Trifluormethyl oder geradkettiges oder verzweigtes Alkyl mit bis zu 14 Kohlenstoffatomen bedeutet, das gegebenenfalls bis zu 2-fach durch Phenyl, Naphthyl oder Pyridyl substituiert ist, oder
- Phenyl oder Naphthyl bedeutet, das gegebenenfalls durch Fluor, Chlor, Trifluormethyl, Trifluormethoxy oder durch geradkettiges oder verzweigtes Alkyl mit bis zu 4 Kohlenstoffatomen substituiert ist,
- Cyclopropyl, Cyclopentyl, Cyclohexyl, Chinolyl, Chinolyl-N-oxid, Indolyl, Pyridyl, Pyridyl-N-oxid, Morpholino oder Piperazinyl bedeutet, oder
- einen Rest der Formel

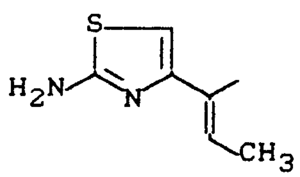
,
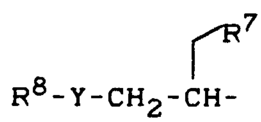
oder

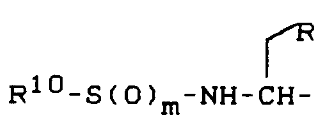

bedeutet,

worin

Y - die CO- oder $SO_2$-Gruppe bedeutet,

$R^7$ - Phenyl oder Naphthyl bedeutet,

$R^8$ und $R^{10}$ gleich oder verschieden sind und geradkettiges oder verzweigtes Alkyl mit bis zu 4 Kohlenstoffatomen, Tolyl, Phenyl oder Naphthyl bedeuten,

m - eine Zahl 1 oder 2 bedeutet,

$R^4$ und $R^5$ gleich oder verschieden sind und

- Wasserstoff oder
- Phenyl oder Naphthyl bedeuten, das gegebenenfalls durch Methyl, Fluor oder Chlor substituiert ist,
- Cyclopropyl, Cyclopentyl oder Cyclohexyl bedeuten, oder

- geradkettiges oder verzweigtes Alkyl mit bis zu 14 Kohlenstoffatomen bedeuten,

$R^6$ - geradkettiges oder verzweigtes Alkyl mit bis zu 4 Kohlenstoffatomen bedeutet,

A, B und D gleich oder verschieden sind und

- für eine direkte Bindung stehen, oder
- für Prolin stehen, oder
- für einen Rest der Formel

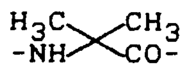

stehen,

- für eine Gruppe der Formel

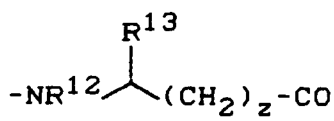

stehen,

worin

z - die Zahl 0 oder 1 bedeutet,

$R^{12}$ - Wasserstoff oder Methyl bedeutet,

$R^{13}$

- Cyclopentyl, Cyclohexyl oder Wasserstoff bedeutet,
- oder geradkettiges oder verzweigtes Alkyl mit bis zu 4 Kohlenstoffatomen bedeutet, das gegebenenfalls durch Methylthio, Hydroxy, Mercapto, Guanidyl, Amino, Carboxy oder $H_2N$-CO- substituiert ist,

  oder durch Cyclohexyl, Naphthyl oder Phenyl substituiert ist, das seinerseits durch Fluor, Chlor oder Alkoxy mit bis zu 4 Kohlenstoffatomen substituiert sein kann, oder

  durch Indolyl, Imidazolyl, Triazolyl, Pyridyl oder Pyrazolyl substituiert ist, wobei die NH-Funktion gegebenenfalls durch Methyl, Benzyloxymethylen oder t-Butyloxycarbonyl (Boc) geschützt ist,

$R^1$ - für geradkettiges oder verzweigtes Alkyl mit bis zu 6 Kohlenstoffatomen steht, das gegebenenfalls durch Cyclopropyl, Cyclopentyl, Cyclohexyl oder Phenyl substituiert ist, das seinerseits durch Hydroxy substituiert sein kann,

n - für die Zahl 1 oder 2 steht, $R^2$ und $R^{2'}$ gleich oder verschieden sind und

- für Hydroxy oder geradkettiges oder verzweigtes Alkoxy mit bis zu 4 Kohlenstoffatomen stehen,

G - für eine Gruppe der Formel -SH oder -OH steht

und

L - für die $-CH_2$ oder -CO-Gruppe steht

und deren physiologisch unbedenklichen Salze.

**4.** Verfahren zur Herstellung der Verbindungen der allgemeinen Formel

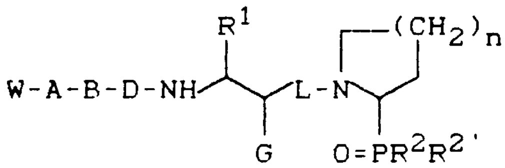

(I)

in welcher

W, A, B, D, G, $R^1$, $R^2$, $R^{2'}$, L und n die oben angegebene Bedeutung haben,

dadurch gekennzeichnet, daß man

[A] Verbindungen der allgemeinen Formel (Ia) oder (Ib)

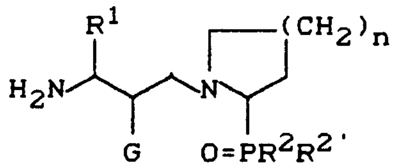

(Ia)

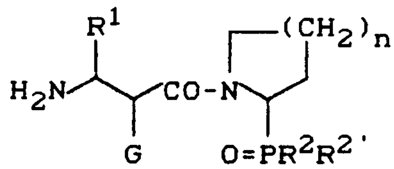

(Ib)

in welcher

$R^1$, $R^2$, $R^{2'}$, G und n die oben angegebene Bedeutung haben,

über die entsprechenden Salze, vorzugsweise über die Hydrochloride,

entweder mit Verbindungen der allgemeinen Formel (II)

W-A'-B'-D'-OH (II)

in welcher

W die oben angegebene Bedeutung hat,

und

A', B' und D' jeweils die oben angegebene Bedeutung von A, B und D haben aber nicht gleichzeitig für eine Bindung stehen,

unter Aktivierung der Carbonsäure, gegebenenfalls in Anwesenheit einer Base und eines Hilfsstoffes, in einem Schritt oder sukzessive (je nach Bedeutung der Substituenten A', B' und D'),

oder mit Verbindungen der allgemeinen Formel (III) oder (IV)

W-X (III)

$(W')_2O$ (IV)

in welcher

W die oben angegebene Bedeutung hat

X für Halogen, vorzugsweise für Chlor steht,

und

W' für die Gruppe $CF_3CO$ oder $CH_3CO$ steht,

nach den in der Peptidchemie üblichen Bedingungen, in inerten Lösungsmitteln, in Anwesenheit einer Base kondensiert,

oder
[B] im Fall, daß L für die $-CH_2$-Gruppe steht, Verbindungen der allgemeinen Formel (V) oder (VI)

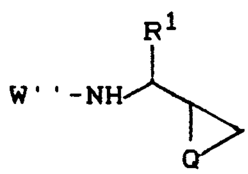
(V)

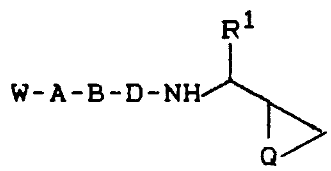
(VI)

in welcher

$R^1$, W, A, B und D die oben angegebene Bedeutung haben

Q - für Sauerstoff oder Schwefel steht

und

W'' für eine Aminoschutzgruppe, vorzugsweise für BOC steht,

mit Verbindungen der allgemeinen Formel (VII)

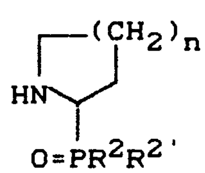
(VII)

in welcher

$R^2$, $R^{2'}$ und n die oben angegebene Bedeutung haben,

in inerten Lösungsmitteln, gegebenenfalls unter Druck umsetzt und im Fall der Verbindungen der allgemeinen Formel (V) die Schutzgruppe W'' anschließend nach üblicher Methode abspaltet und gegebenenfalls mit den Verbindungen der allgemeinen Formel (II) nach der unter Verfahren [A] beschriebenen Methode weiter umsetzt,

oder
[C] im Fall, daß L für die CO-Gruppe steht,

Verbindungen der allgemeinen Formel (VIII)

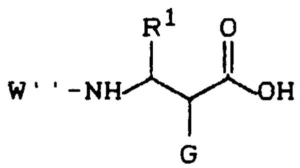

(VIII)

in welcher

W'', G und $R^1$ die oben angegebene Bedeutung haben,

mit den Verbindungen der allgemeinen Formel (VII) unter Aktivierung der Carbonsäure in Anwesenheit einer Base und eines Hilfsstoffes umsetzt und gegebenenfalls unter Abspaltung der Schutzgruppe W'' die Gruppe W-A'-B'-D'-OH nach der unter [A] angegebenen Methode einführt.

**5.** Verfahren zur Herstellung der Verbindungen der allgemeinen Formel (VIa)

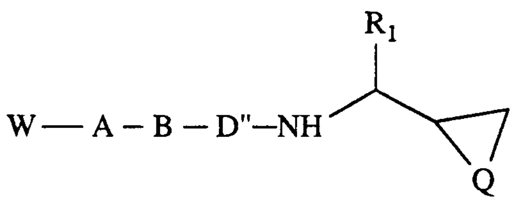

in welcher

W, A, B, $R^1$ und Q die oben angegebene Bedeutung haben,

D'' die Bedeutung von D hat mit der Ausnahme einer direkten Bindung

dadurch gekennzeichnet, daß man
[D] im Fall, daß Q für ein Sauerstoffatom steht, Verbindungen der Formel (IX)

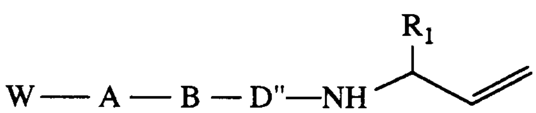

(IX)

in welcher

W, A, B, D'' und $R^1$ die oben angegebene Bedeutung haben,

in inerten Lösemitteln mit einer Epoxidierungsreaktion, gegebenenfalls mit Hilfe einer Base oder einer Phasentransferkatalyse zu Verbindungen der allgemeinen Formel (VIb)

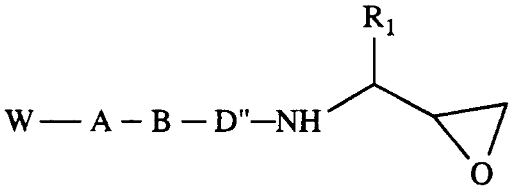

(VIb)

in welcher

W, A, B, D'' und $R^1$ die oben angegebene Bedeutung haben,

umsetzt, und
[E] im Fall, daß Q für ein Schwefelatom steht, die Verbindungen der allgemeinen Formel (VIb) mit Thiodimethylformamid der Formel (X)

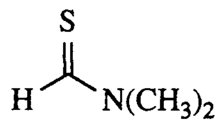

(X)

in inerten Lösemitteln, in Anwesenheit von Säuren, in einer Umlagerungsreaktion zu Verbindungen der allgemeinen Formel (VIc)

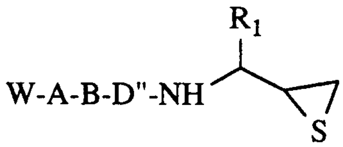

(VIc)

in welcher

W, A, B, D'' und $R^1$ die oben angegebene Bedeutung haben,

weiter umsetzt.

**6.** Verbindungen der allgemeinen Formel (VIa)

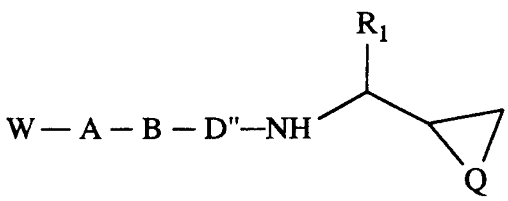

(VIa)

in welcher

W, A, B, $R^1$ und Q die in Anspruch 1 angegebene Bedeutung haben

und

D'' mit Ausnahme einer Bindung die Bedeutung von D aus Anspruch 1 haben kann.

**7.** Arzneimittel enthaltend eine oder mehrere Verbindungen der Ansprüche 1 bis 3 und 6.

**8.** Antivirale Mittel enthaltend eine oder mehrere Verbindungen der Ansprüche 1 bis 3 und 6.

**9.** Verwendung der Verbindungen der Ansprüche 1 bis 3 und 6 zur Herstellung von antiviralen Arzneimitteln.